# EUROPÄISCHE PATENTSCHRIFT

(11) **EP 1 844 039 B1**
(45) Veröffentlichungstag und Bekanntmachung des Hinweises auf die Patenterteilung: **15.07.2009**
(21) Anmeldenummer: 05808294.2
(22) Anmeldetag: 11.11.2005
(51) Int. Cl.: C07D 405/12, C07D 307/79, C07D 307/83, C07C 69/68, A61K 31/47, A61P 29/00

(54) **5-SUBSTITUIERTE CHINOLIN- UND ISOCHINOLIN-DERIVATE; EIN VERFAHREN ZU IHRER HERSTELLUNG UND IHRE VERWENDUNG ALS ENTZÜNDUNGSHEMMER**
5-SUBSTITUTED QUINOLINE AND ISOQUINOLINE DERIVATIVES, A METHOD FOR THE PRODUCTION THEREOF AND THEIR USE AS ANTIPHLOGISTICS
DERIVES DE QUINOLINE ET D'ISOQUINOLINE 5-SUBSTITUES, PROCEDE PERMETTANT DE LES PRODUIRE ET LEUR UTILISATION COMME ANTIPHLOGISTIQUE

(30) Priorität: 12.11.2004 DE 102004055633; 26.04.2005 DE 102005020331
(43) Veröffentlichungstag der Anmeldung: 17.10.2007
(73) Patentinhaber: Bayer Schering Pharma Aktiengesellschaft, 13353 Berlin (DE)
(72) Erfinder: JAROCH, Stefan, 10629 BERLIN Berlin (DE); REHWINKEL, Hartmut, 10961 Berlin (DE); SCHÄCKE, Heike, 10115 Berlin (DE); SCHMEES, Norbert, 13469 Berlin (DE); SKUBALLA, Werner, 13465 Berlin (DE); SCHNEIDER, Matthias, 10589 Berlin (DE); HÜBNER, Jan, 10317 Berlin (DE); PETROV, Orlin, 14195 Berlin (DE); DINTER, Christian, 13189 Berlin (DE)
(74) Vertreter: Seuss, Thomas
(86) Internationale Anmeldenummer: PCT/EP2005/012531
(87) Internationale Veröffentlichungsnummer: WO 2006/050998

(56) Entgegenhaltungen:
- WO-A-03/082827
- WO-A-20/04063163
- WO-A-20/04075864
- WO-A-20/05090343

## Beschreibung

Die Erfindung betrifft 5-substituierte Chinolin- und Isochinolin-Derivate, ein Verfahren zu ihrer Herstellung und ihre Verwendung als Entzündungshemmer.

Aus dem Stand der Technik WO03/082827 sind Entzündungshemmer der allgemeinen Formel 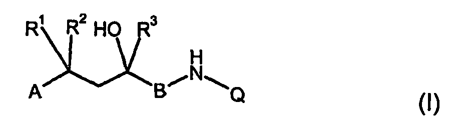 bekannt, wobei der Q-Rest Chinolin- und Isochinolin-Derivate umfaßt. Diese Verbindungen zeigen im Experiment Wirkdissoziationen zwischen antiinflammatorischen und unerwünschten metabolischen Wirkungen und sind den bisher beschriebenen, nichtsteroidalen Glucocorticoiden überlegen oder weisen zumindest eine ebenso gute Wirkung auf. Zudem weisen diese Verbindungen eine verbesserte Selektivität gegenüber anderen Steroidrezeptoren auf.

WO 2004/063163 A1 (Boehringer Ingelheim Pharmaceuticals) beschreibt Glukokortikoid-Liganden mit ähnlicher Struktur, bei der jedoch die typische N-H-Gruppe der Verbindungen der Formel I der WO 03/082827 und der erfindungsgemäßen Verbindungen fehlen. Die Verbindungen der WO 20041063163 werden als Glukokortikoid-Liganden beschrieben, experimentelle Daten zur Bindung an den Glukokortikoidrezeptor werden allerdings nicht offenbart.

Überraschenderweise wurden nun gefunden, daß Verbindungen der Formeln (IIa) und (IIb) besonders aktiv und dissoziiert im Hinblick auf Nebenwirkungen sind und bevorzugt für eine lokale Applikation infrage kommen.

Die vorliegende Erfindung betrifft daher Verbindungen der allgemeinen Formeln (IIa) und (IIb) 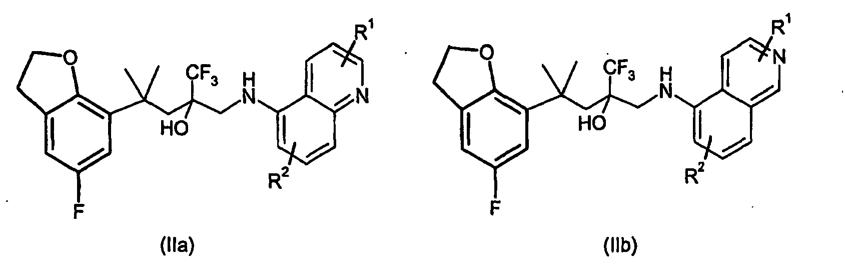 worin
- R¹ und R²: unabhängig voneinander ein Wasserstoffatom, eine C₁₋₃-Alkylgruppe, ein Halogenatom, eine Cyano-, eine C₁₋₃-Alkoxy- oder eine Hydroxygruppe sein können,
sowie deren Racemate oder getrennt vorliegenden Stereoisomere und gegebenenfalls deren physiologisch verträgliche Salze.

Die Bezeichnung Halogenatom oder Halogen bedeutet ein Fluor-, Chlor-, Brom- oder lodatom. Bevorzugt ist ein Fluor-, Chlor oder Bromatom.

Die C₁-C₃-Alkylgruppen und die C₁-C₅-Alkylgruppen können geradkettig oder verzweigt sein und für eine Methyl-, Ethyl-, n-Propyl-, iso-Propyl-, n-Butyl, iso-Butyl, tert.-Butyl- oder n-Pentyl-, 2,2-Dimethylpropyl-, 2-Methylbutyl- oder 3-Methylbutylgruppe stehen.
Eine Methyl- oder Ethylgruppe ist bevorzugt.

Die Reste R¹ und R² bedeuten bevorzugt Wasserstoff, C₁₋₃-Alkyl, Halogen oder Hydroxy. Besonders bevorzugt sind Wasserstoff, Methyl, Chlor und Hydroxy.

Somit betrifft ein besonderer Gegenstand der Erfindung Verbindungen der allgemeinen Formel IIa und IIb, worin R¹ und R² unabhängig voneinander bevorzugt Wasserstoff, C₁₋₃-Alkyl, Halogen oder Hydroxy bedeuten.

Besonders bevorzugt sind Verbindungen der Formel IIa und IIb, worin R¹ und R² unabhängig voneinander Wasserstoff, Methyl, Chlor oder Hydroxy bedeuten.

Ein besonderer Aspekt der vorliegenden Erfindung sind die Verbindungen der allgemeinen Formel IIa.

Der Begriff "lokal" umfaßt jegliche mögliche Verabreichung der erfindungsgemäßen Verbindungen, die eine direkte Penetration des Wirkstoffes an den Wirkort ermöglicht.

Die erfindungsgemäßen Verbindungen der allgemeinen Formel (IIa) und (IIb) können durch das Vorhandensein von Asymmetriezentren als unterschiedliche Stereoisomere vorliegen. Sowohl die Racemate als auch die getrennt vorliegenden Stereoisomere gehören zum Gegenstand der vorliegenden Erfindung.

Ein besonderer Gegenstand der vorliegenden Erfindung sind die getrennt vorliegenden Stereoisomere, d.h. (+)-Enantiomere und (-)-Enantiomere, insbesondere der Beispiele 1, 2, 3, 4, 5, 11 und 12.

Ferner zeichnen sich die erfindungsgemäßen Verbindungen, wenn sie eine Hydroxygruppe in α-Position zum Chinolinyl- oder Isochinolinyl-Stickstoffatom enthalten, durch das Vorliegen einer Keto-Enol-Tautomerie aus. Im erfindungsgemäßen Sinne gehören beide Formen zum Gegenstand der Erfindung, selbst wenn, z.B. im experimentellen Teil, nur eine der beiden tautomeren Formen aufgeführt worden ist.

Insbesondere sind Gegenstand der vorliegenden Erfindung:
5-[4-(5-Fluor-2,3-dihydrobenzofuran-7-yl)-2-hydroxy-4-methyl-2-trifluormethyl-pentylamino]-2-methylchinolin),
5-[4-(5-Fluor-2,3-dihydrobenzofuran-7-yl)-2-hydroxy-4-methyl-2-trifluormethyl-pentylamino]-1-methylisochinolin),
5-[4-(5-Fluor-2,3-dihydrobenzofuran-7-yl)-2-hydroxy-4-methyl-2-trifluormethyl-pentylamino]isochinol-1(2H)-on,
5-[4-(5-Fluor-2,3-dihydrobenzofuran-7-yl)-2-hydroxy-4-methyl-2-trifluormethyl-pentylamino]-2,6-dimethylchinolin,
5-[4-(5-Fluor-2,3-dihydrobenzofuran-7-yl)-2-hydroxy-4-methyl-2-trifluormethyl-pentylamino]-6-chlor-2-methylchinolin,
5-[4-(5-Fluor-2,3-dihydrobenzofuran-7-yl)-2 -hydroxy-4-methyl-2-trifluormethyl-pentylamino]isochinolin,
5-[4-(5-Fluor-2,3-dihydrobenzofuran-7-yl)-2-hydroxy-4-methyl-2-trifluormethyl-pentylamino]chinolin,
5-[4-(2,3-Dihydro-5-fluoro-7-benzofuranyl)-2-hydroxy-4-methyl-2-trifluormethyl-pentylamino]chinolin-2[1H]-on,
6-Fluor-5-[4-(5-fluor-2,3-dihydrobenzofuran-7-yl)-2-hydroxy-4-methyl-2-trifluormethyl-pentylamino]-2-methylchinolin,
8-Fluor-5-[4-(5-fluor-2,3-dihydrobenzofuran-7-yl)-2-hydroxy-4-methyl-2-trifluormethyl-pentylamino]-2-methylchinolin,
5-[4-(5-Fluor-2,3-dihydrobenzofuran-7-yl)-2-hydroxy-4-methyl-2-trifluormethyl-pentylamino]-2-methylisochinol-1(2H)-on sowie ihre getrennten Enantiomere:
2(R)-5-[4-(5-Fluor-2,3-dihydrobenzofuran-7-yl)-2-hydroxy-4-methyl-2-trifluormethyl-pentylamino]-2-methylchinolin),
2(R)-5-[4-(5-Fluor-2,3-dihydrobenzofuran-7-yl)-2-hydroxy-4-methyl-2-trifluormethyl-pentylamino]-1-methylisochinolin),
2(R)-5-[4-(5-Fluor-2,3-dihydrobenzofuran-7-yl)-2-hydroxy-4-methyl-2-trifluormethyl-pentylamino]isochinol-1(2H)-on,
2(R)-5-[4-(5-Fluor-2,3-dihydrobenzofuran-7-yl)-2-hydroxy-4-methyl-2-trifluormethyl-pentylamino]-2,6-dimethylchinolin,
2(R)-5-[4-(5-Fluor-2,3-dihydrobenzofuran-7-yl)-2-hydroxy-4-methyl-2-trifluormethyl-pentylamino]-6-chlor-2-methylchinolin,
2(R)-5-[4-(5-Fluor-2,3-dihydrobenzofuran-7-yl)-2-hydroxy-4-methyl-2-trifluormethyl-pentylamino]isochinolin,
2(R)-5-[4-(5-Fluor-2,3-dihydrobenzofuran-7-yl)-2-hydroxy-4-methyl-2-trifluormethyl-pentylamino]chinolin,
2(R)-5-[4-(2,3-Dihydro-5-fluoro-7-benzofuranyl)-2-hydroxy-4-methyl-2-trifluormethyl-pentylamino]chinolin-2[1H]-on,
2(R)-6-Fluor-5-[4-(5-fluor-2,3-dihydrobenzofuran-7-yl)-2-hydroxy-4-methyl-2-trifluormethyl-pentylamino]-2-methylchinolin,
2(R)-8-Fluor-5-[4-(5-fluor-2,3-dihydrobenzofuran-7-yl)-2-hydroxy-4-methyl-2-trifluormethyl-pentylamino]-2-methylchinolin,
2(R)-5-[4-(5-Fluor-2,3-dihydrobenzofuran-7-yl)-2-hydroxy-4-methyl-2-trifluormethyl-pentylamino]-2-methylisochinol-1(2H)-on.
2(S)-5-[4-(5-Fluor-2,3-dihydrobenzofuran-7-yl)-2-hydroxy-4-methyl-2-trifluormethyl-pentylamino]-2-methylchinolin),
2(S)-5-[4-(5-Fluor-2,3-dihydrobenzofuran-7-yl)-2-hydroxy-4-methyl-2-trifluormethyl-pentylamino]-1-methylisochinolin),
2(S)-5-[4-(5-Fluor-2,3-dihydrobenzofuran-7-yl)-2-hydroxy-4-methyl-2-trifluormethyl-pentylamino]isochinol-1(2H)-on,
2(S)-5-[4-(5-Fluor-2,3-dihydrobenzofuran-7-yl)-2-hydroxy-4-methyl-2-trifluormethyl-pentylamino]-2,6-dimethylchinolin,
2(S)-5-[4-(5-Fluor-2,3-dihydrobenzofuran-7-yl)-2-hydroxy-4-methyl-2-trifluormethyl-pentylamino]-6-chlor-2-methylchinolin,
2(S)-5-[4-(5-Fluor-2,3-dihydrobenzofuran-7-yl)-2-hydroxy-4-methyl-2-trifluormethyl-pentylamino]isochinolin,
2(S)-5-[4-(5-Fluor-2,3-dihydrobenzofuran-7-yl)-2-hydroxy-4-methyl-2-trifluormethyl-pentylamino]chinolin,
2(S)-5-[4-(2,3-Dihydro-5-fluoro-7-benzofuranyl)-2-hydroxy-4-methyl-2-trifluormethyl-pentylamino]chinolin-2[1H]-on,
2(S)-6-Fluor-5-[4-(5-fluor-2,3-dihydrobenzofuran-7-yl)-2-hydroxy-4-methyl-2-trifluormethyl-pentylamino]-2-methylchinolin,
2(S)-8-Fluor-5-[4-(5-fluor-2,3-dihydrobenzofuran-7-yl)-2-hydroxy-4-methyl-2-trifluormethyl-pentylamino]-2-methylchinolin,
2(S)-5-[4-(5-Fluor-2,3-dihydrobenzofuran-7-yl)-2-hydroxy-4-methyl-2-trifluormethyl-pentylamino]-2-methylisochinol-1(2H)-on.

Besonders bevorzugt ist 5-[4-(5-Fluor-2,3-dihydrobenzofuran-7-yl)-2-hydroxy-4-methyl-2-trifluormethyl-pentylamino]-2-methylchinolin, und dessen getrennt vorliegende Enantiomere 2-(R)-5-[4-(5-Fluor-2,3-dihydrobenzofuran-7-yl)-2-hydroxy-4-methyl-2-trifluormethyl-pentylamino]-2-methylchinolin und 2-(S)-5-[4-(5-Fluor-2,3-dihydrobenzofuran-7-yl)-2-hydroxy-4-methyl-2-trifluormethyl-pentylamino]-2-methylchinolin.

Die Verfahren zur Herstellung der Verbindungen aus WO98/54159, WO00/32584 und WO02/10143 können auch für die Herstellung der erfindungsgemäßen Verbindungen verwendet werden. Für die Anknüpfung der für die erfindungsgemäßen Verbindungen charakteristischen Chinolin- oder Isochinolingruppe können folgende Verfahrensschritte durchgeführt werden:

Die Titelverbindungen (IIa) und (IIb) lassen sich beispielsweise durch reduktive Aminierung der Verbindung der Formel (III) mit 5-Aminochinolinen bzw. 5-Aminoisochinolinen synthetisieren, wobei z.B Natriumborhydrid oder Natriumcyanoborhydrid in Gegenwart einer Säure als Reduktionsmittel in Betracht kommen.

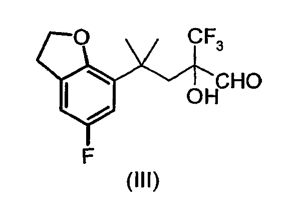

Die Synthese des Aldehyds gelingt beispielsweise ausgehend von Verbindung (IV) (WO0032584) durch Spaltung des Methylethers, Allylierung des resultierenden Phenols (V), Umlagerung der Allylethers (VI) zu (VII), Dihydroxylierung und Glykolspaltung der Doppelbindung unter Bildung des Lactols (VIII), Reduktion des Lactols zum Diol (IX), Ringschluß zum Dihydrobenzofuran (X), Reduktion des Esters zu Alkohol (XI), der schließlich zum Aldehyd (III) oxidiert wird.

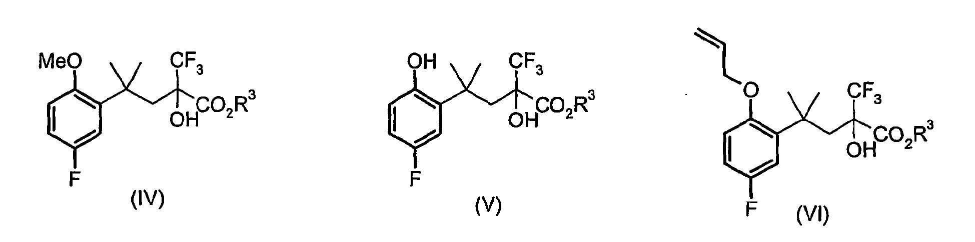

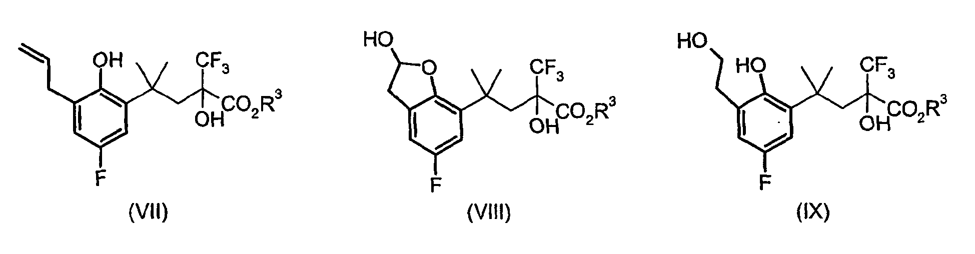

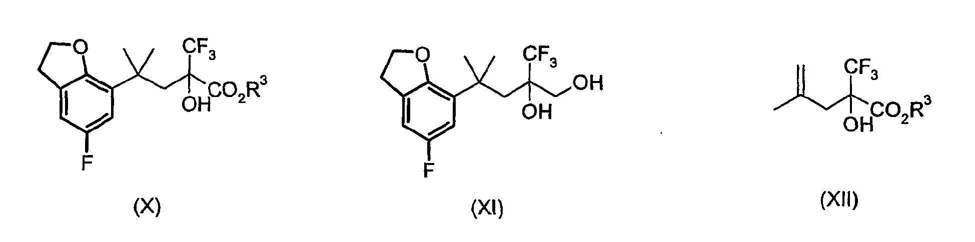

Die oben aufgeführten Ester sind vorzugsweise Ethylester, können jedoch Ester vom Typ -COOR³ sein, wobei R³ C₁-C₅Alkyl bedeutet.

Ein besonderer Gegenstand der Erfindung ist die Herstellung der Verbindungen der allgemeinen Formel (IIa) und (IIb) indem der Aldehyd (III) in chiraler oder racemischer Form unter Bedingungen der reduktiven Aminierung, gegebenenfalls in zwei Stufen, mit einem 5-Aminochinolinderivat oder einem 5-Aminoilsochinolinderivat 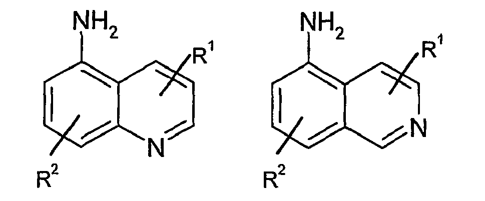 umgesetzt wird, wobei R¹ und R² die für die Verbindungen der Formel IIa und IIb in Anspruch 1 angegebenen Bedeutungen haben.

Ein weiterer Gegenstand der Erfindung ist die Herstellung des Aldehyds (III) , der durch Reduktion der Verbindung der allgemeinen Formel X - eingesetzt als chirale Verbindung oder als Racemat - worin R³ C₁-C₅-Alkyl bedeutet, nach dem Fachmann bekannten Methoden zum Alkohol (XI) und anschließender Oxidation nach ebenfalls dem Fachmann Methoden zum Aldehyd oder durch gebremste Reduktion nach dem Fachmann bekannter Methode vom Ester X direkt zum Aldehyd erhalten werden kann.

Ein weiterer Gegenstand der Erfindung ist das Verfahren zur Herstellung der Verbindung der allgemeinen Formel X, die durch Friedel Crafts Alkylierung von 5-Fluoro-2,3-dihydrobenzofuran erhalten werden und anschließend einer Enantiomerentrennung unterworfen werden kann.

Ein alternatives Verfahren zur Herstellung der Verbindungen der Formel IIa und IIb wird wie folgt durchgeführt:

Isobuten wird unter Verwendung eines lewissauren Katalysators, beispielsweise TiCl₄, Ti(OR³)₄, TiCl₂(OR³)₂, TiBr₂(C)R)₂, PdCl₄, Pd(OR³)₄, PdCl₂(OR³)₂, PdBr₂(OR³)₂, ZnCl₂, ZnBr₂, AlCl₃, AlBr₃, AlEtCl₂, Al Me₂Cl, Cu-Salze z. B. Cu(OTf)₂, CuCl₂, CuBr₂, Yb(OTf)₃, chiralen Katalysatoren wie z.B. (BINOL)₂TiCl₂, (BINAP)₂TiCl₂, (BINOL)₂PdCl₂, (BINAP)₂PdCl₂, (BINOL)₂TiBr₂, (BINAP)₂TiBr₂, (BINOL)₂PdBr₂, (BINAP)₂PdBr₂, bevorzugt FeCl₃, wobei R³ C₁C₅-Alkyl bedeutet,
mit Trifluorethylpyruvat zum 2-Hydroxy-4-methyl-2-trifluormethylpent-4-ensäureethylester XII umgesetzt. Dieses Reaktionsprodukt wird anschließend in einem weiteren Reaktionsschritt mit 5-Fluor-2,3-dihydrobenzofuran umgesetzt, um eine Verbindung der Formel (X) zu erhalten. Reduktion des Esters zum Alkohol (XI) und anschließende Oxidation zum Aldehyd (III) oder Reduktion vom Ester (X) zum Aldehyd (III) nach für den Fachmann üblichen Methoden erschließen mit dem Aldehyd die direkte Vorstufe für die Verbindungen der allgemeinen Formel IIa und IIb, die dann durch Umsetzung des Aldehyds (III) mit dem entsprechenden Chinolinamin oder Isochinolinamin unter Bedingungen der reduktiven Aminierung , wie bereits im Stand der Technik beschrieben, erhalten werden können. Auf der Stufe des Esters (XII) oder des Esters (X) kann eine Enantiomerentrennung durchgeführt werden. Auch der Alkohol (XI) ist für eine Enantiomerentrennung geeignet. Ein getrenntes Einsetzen der chiralen Ester führt dann zu den enantiomerenreinen Verbindungen der allgemeinen Formel IIa und IIb.

Ein weiterer Gegenstand der Erfindung ist ein Verfahren zur Herstelllung von Verbindungen der allgemeinen Formel XII, worin R³ C₁-C₅-Alkyl bedeutet, indem Isobuten unter Verwendung eines lewissauren Katalysators mit Trifluoralkylpyruvat umgesetzt wird und das Reaktionsprodukt in die Enantiomeren getrennt wird.

Als Lösungsmittel für die Reaktion von Isobuten mit Trifluorethylpyruvat sind beispielsweise CH₂Cl₂, Tetrahydrofuran, Dioxan, Diethylether geeignet.

Ein besonderer Aspekt der Erfindung ist die Verwendung von chiralen lewissauren Katalysatoren für das erfindungsgemäße Verfahren.

Ein Aspekt oder Erfindung ist, dass die Enantiomerentrennung auf einer beliebigen Stufe der Synthese mit Hilfe von Säulenchromatographie an einer chiralen Phase erfolgt. Die Trennung auf der Stufe der Verbindung der Formel IIa oder IIb ist ein besonderer Aspekt der Erfindung. Die Enantiomerentrennung auf der Stufe der Ester XII oder X ist ein weiterer wichtiger Aspekt der Erfindung.

Ein weiterer Aspekt der Erfindung ist die Trennung von geeigneten racemischen Zwischenstufen der Synthese mit Hilfe von chiralen Hilfsstoffen. Die racemischen Zwischenstufen können entweder mit chiralen Hilfsstoffen z.B. Basen in diastereomere Salze überführt werden oder mit chiralen Hilfsstoffen in Diastereomere überführt werden, die anschließend einer Diastereomerentrennung unterworfen werden. Das chirale Hilfsreagenz wird dann wieder abgespalten und kann zurückgewonnen werden.

Geeignete chirale Hilfsreagenzien sind dem Fachmann bekannt und beispielsweise dem Buch "Chiral Auxiliaries and Ligands in Asymmetric Synthesis", von J. Seyden-Penne, Wiley Verlag, New York (1995) zu entnehmen.

Geeignete Zwischenstufen zur Trennung von racemischen Zwischenstufen sind beisielsweise:
a) alle Vorstufen, die mindestens eine Alkoholfunktion aufweisen; als Hilfsreagenzien sind hierfür chirale Säuren geeignet.
b) Aldehydvorstufen, wie z.B. der Aldehyd (III), der nach Reduktion des Esters zum Alkohol (XI) und anschließende Oxidation zum Aldehyd (III) oder Reduktion vom Ester (X) zum Aldehyd (III) erhalten wird; gegebenenfalls können auch andere Estervorstufen extra zur Enantiomerentrennung in die Aldehyde überführt werden.
   Hilfsreagenzien sind hierfür chirale Diole, die dann diastereomere Ketale bilden, die getrennt und anschließend wieder gespalten werden können.
c) alle Säuren, die gegebenenfalls aus den Estervorstufen durch Spaltung der vorhandenen Esterfunktion oder durch Oxidation von Verbindungen niedrigerer Oxidationsstufen nach dem Fachmann bekannten Methoden erhalten werden können;
   als Hilfsreagenzien können hier chirale Alkohole oder chirale Amine verwendet werden.
d) alle Vorstufen, die eine Esterfunktion enthalten, können über Umesterung in diastereomere Ester überführt werden, die dann wie unter c) beschrieben behandelt werden können.

Wenn die erfindungsgemäßen Verbindungen als racemische Gemische vorliegen, können sie nach dem Fachmann geläufigen Methoden der Racemattrennung in die reinen, optisch aktiven Formen aufgetrennt werden. Beispielsweise lassen sich die racemischen Gemische durch Chromatographie an einem selbst optisch aktiven Trägermaterial (z.B. CHIRALPAK AD^{®}) in die reinen Isomere trennen. Geeignete Vorstufen sind Verbindungen der allgemeinen Formel III, IV, V, VI, VII, VIII, IX, X, XI.

Es ist auch möglich, die freie Hydroxygruppe in einer racemischen Verbindung der allgemeinen Formel (IIa) und (IIb) oder einer geeigneten Vorstufe mit einer optisch aktiven Säure zu verestern und die erhaltenen diastereoisomeren Ester durch fraktionierte Kristallisation oder chromatographisch zu trennen und die getrennten Ester jeweils zu den optisch reinen Isomeren zu verseifen. Als optisch aktive Säure kann beispielsweise Mandelsäure, Camphersulfonsäure oder Weinsäure verwendet werden. Wie dem Fachmann bekannt, kann das Hilfreagenz gegebenenfalls zurückgewonnen werden.

Somit ist ein besonderer Gegenstand der Erfindung ein Verfahren wie in einem der beiden oben beschriebenen Alternativen dargestellt, das dadurch gekennzeichnet ist, dass die Diastereomerentrennung auf einer beliebigen geeigneten Stufe durch Verestern einer Alkoholfunktion mit einer chiralen Säure, Auftrennen der Diastereomeren und Verseifen mit oder ohne Rückgewinnung des chiralen Hilfsreagenzes vorgenommen wird.

Im Falle, daß die Verbindungen der allgemeinen Formel (IIa) und (IIb) als Salze vorliegen, kann dies beispielsweise in der Form des Hydrochlorids, Sulfats, Nitrats, Phosphats, Pivalats, Maleats, Fumarats, Tartrats, Benzoats, Mesylats, Citrats oder Succinats sein.

Die Bindung der Substanzen an den Glucocorticoid-Rezeptor (GR) und weitere Steroidhormon-Rezeptoren (Mineralcorticoid-Rezeptor (MR), Progesteron-Rezeptor (PR) und Androgen-Rezeptor (AR)) wird mit Hilfe rekombinant hergestellter Rezeptoren überprüft. Die Bindungsexperimente werden mit Extrakten aus Sf9 Zellen, die mit Baculoviren, welche die codierenden Sequenzen für den jeweiligen Steroidhormon-Rezeptor enthalten, infiziert waren. Im Vergleich zur Bezugssubstanz [³H]-Dexamethason zeigen die Substanzen eine hohe bis sehr hohe Affinität zum GR.

Darüberhinaus zeigen die hier beschriebenen Chinoline und Isochinoline der Formel (IIa) und (IIb) eine hohe Selektivität für den Glucocorticoid-Rezeptor.

Als wesentlicher, molekularer Mechanismus für die anti-entzündliche Wirkung von Glucocorticoiden wird die durch den GR vermittelte Hemmung der Transkription von Zytokinen, Adhäsionsmolekülen, Enzymen und anderer pro - inflammatorischen Faktoren angesehen. Diese Hemmung wird durch eine Interaktion des GR mit anderen Trankriptionsfaktoren, z.B. AP-1 und NF-kappa-B, bewirkt (zur Übersicht siehe Cato, ACB and Wade E, BioEssays 18, 371-378 1996).

Die erfindungsgemäßen Verbindungen der allgemeinen Formel (IIa) und (IIb) hemmen die durch Lipopolysaccharid (LPS) induzierte Sekretion des Zytokins IL-8 in der menschlichen Monozytenzelline THP-1. Die Konzentration der Cytokine wurde im Überstand mittels kommerziell erhältlicher ELISA-Kits bestimmt (Efficacy von Dexamethason = 100%): Beispiel 1, IC₅₀ = 5.9 nM (74% Efficacy); Beispiel 10, IC₅₀ = 21nM (86% Efficacy); Beispiel 11, IC₅₀ = 8.5 nM (61 % Efficacy); Prednisolon, IC₅₀ = 13 nM (96% Efficacy).

Die anti - entzündliche Wirkung der Verbindungen der allgemeinen Formel (IIa) und (IIb) wurden im Tierexperiment durch Testen in der Crotonöl - induzierten Entzündung in der Ratte und / oder der Maus getestet (J. Exp. Med. (1995), 182, 99-108). Hierzu wurde den Tieren Crotonöl in ethanolischer Lösung topisch auf die Ohren appliziert. Die Testsubstanzen wurden gleichzeitig lokal appliziert. Nach 16-24 Stunden wurden das Ohrgewicht als Maß für das entzündliche Ödem, die Peroxidaseaktivität als Maß für die Einwanderungen von Granulozyten und die Elastaseaktivität als Maß für die Einwanderung von neutrophilen Granulozyten gemessen. Die Verbindungen der allgemeinen Formel (IIa) und (IIb) hemmen in diesem Test nach topischer Applikation die drei oben genannten Entzündungsparameter.

Eine der häufigsten unerwünschten Wirkungen einer Glucocorticoid - Therapie ist der sogenannte "Steroiddiabetes" [vgl. Hatz, HJ, Glucocorticoide:

Immunologische Grundlagen, Pharmakologie und Therapierichtlinien, Wissenschafliche Verlagsgesellschaft mbH, Stuttgart, 1998]. Ursache hierfür ist die Stimulation der Gluconeogenese in der Leber durch Induktion der hierfür verantwortlichen Enzyme und durch freie Aminosäuren, die aus dem Abbau von Proteinen (katabole Wirkung der Glucocorticoide) entstehen. Ein Schlüsselenzym des katabolen Stoffwechsels in der Leber ist die Tyrosinaminotranferase (TAT). Die Aktivität dieses Enzyms kann photometrisch aus Zellkulturen von behandelten Ratten-Hepatomzellen bestimmt werden. Die Zellen werden für 24 Stunden mit den Testsubstanzen behandelt und anschließend die TAT-Aktivität gemessen. Die Verbindungen der allgemeinen Formel (IIa) und (IIb) induzieren in diesem Test in geringem Maße die Tyrosinaminotransferase (Efficacy von Dexamethason = 100%): Beispiel 1, EC₅₀ = 3.7 nM (93% Efficacy); Beispiel 10, EC₅₀ = 10 nM (92% Efficacy); Beispiel 11, EC₅₀ = 4.0 nM (86% Efficacy); Prednisolon, EC₅₀ = 2.6 nM (103% Efficacy).

Eine weitere, besonders nach lokaler Therapie, auftretende unerwünschte Wirkung ist die Induktion einer Hautatrophie, die zu einem Verlust der Haut an Dicke, Elastizität und letztlich der mechanischen Belastbarkeit der Haut führt. Das Potential einer Substanz, Hautatrophie zu induzieren, kann in Ratten bestimmt werden. Die Tiere werden dafür 18 Tage täglich lokal in äquieffektiven Dosierungen mit den Testsubstanzen behandelt. Mittels einer Hautfaltendickenmessung läßt sich die Abnahme der Hautdicke über die Behandlungszeit verfolgen.

Im Vergleich zu Clobetasolpropionat, das bei einer Konzentration von 0.01 % (maximale anti-inflammatorische Wirkung wird erreicht) zu einer 65%igen Reduktion der Hautdicke führt, ist bei Beispiel 1 bei einer Konzentration von 0.1 % (maximale anti-inflammatorische Wirkung wird erreicht) nur eine 41 %ige Reduktion festzustellen.

Dieser Vorteil ist auch gegenüber den Substanzen aus der Anmeldung WO03/082827 zu sehen; beispielsweise induziert das Eutomer von Beispiel 36 bereits bei einer Konzentration von 0.06% (maximale anti-inflammatorische Wirkung) eine 60%ige Reduktion der Hautdicke.

Aufgrund ihrer anti-entzündlichen und zusätzlichen anti-allergischen, immunsuppressiven und anti-proliferativen Wirkung können die erfindungsgemäßen Verbindungen der allgemeinen Formel (IIa) und (IIb) als Medikamente zur Behandlung oder Prophylaxe folgender Krankheitszustände bei Säugetieren und Menschen, insbesondere für die lokale Applikation Verwendung finden:
Dabei steht der Begriff "ERKRANKUNG" für die folgenden Indikationen:
   (i) Lungenerkrankungen, die mit entzündlichen, allergischen und / oder proliferativen Prozessen einhergehen:
      - Chronisch obstruktive Lungenerkrankungen jeglicher Genese, vor allem Asthma bronchiale
      - Bronchitis unterschiedlicher Genese
      - Adult respiratory distress syndrome (ARDS), akutes Atemnotssyndrom
      - Bronchiektasen
         - Alle Formen der restriktiven Lungenerkrankungen, vor allem allergische Alveolitis,
      - Alle Formen des Lungenödems, vor allem toxisches Lungenödem, z.B. Strahlenpneumonitis
      - Sarkoidosen und Granulomatosen, insbesondere Morbus Boeck
   (ii) Rheumatische Erkrankungen / Autoimmunerkrankungen / Gelenkerkrankungen, die mit entzündlichen, allergischen und / oder proliferativen Prozessen einhergehen:
      - Alle Formen rheumatischer Erkrankungen, insbesondere rheumatoide Arthritis, akutes rheumatisches Fieber, Polymyalgia rheumatica, Morbus Behcet
      - Reaktive Arthritis
      - Entzündliche Weichteilerkrankungen sonstiger Genese
      - Arthritische Symtome bei degenerativen Gelenkerkrankungen (Arthrosen)
      - Traumatische Arthritiden
      - Vitiligo
      - Kollagenosen jeglicher Genese, z.B. systemischer Lupus erythematodes, Sklerodermie, Polymyositis, Dermatomyositis- Sjögren-Syndrom, Still-Syndrom, Felty-Syndrom
      - Sarkoidosen und Granulomatosen
      - Weichteilrheumatismus
   (iii) Allergien oder pseudoallerg. Erkrankungen, die mit entzündlichen, und / oder proliferativen Prozessen einhergehen:
      - Alle Formen allergischer Reaktionen, z.B. Quincke Ödem, Heuschnupfen, Insektenstich, allergische Reaktionen auf Arzneimittel, Blutderivate, Kontrastmittel etc., Anaphylaktischer Schock, Urtikaria, allergische und irritative Kontakdermatitis, allergische Gefäßerkrankungen
      - Vasculitis allergica
   (iv) Gefäßentzündungen (Vaskulitiden)
      - Panarteriitis nodosa, Arteriitis temporalis, Erythema nodosum
      - Polyarteritis nodosa
      - Wegner Granulomatose
      - Riesenzellarteriitis
   (v) Dermatologische Erkrankungen, die mit entzündlichen, allergischen und / oder proliferativen Prozessen einhergehen:
      - Atopische Dermatitis (vor allem bei Kindern)
      - Sämtliche Ekzemformen wie z.B. atopisches Ekzem (v.a. bei Kindern)
      - Exantheme jedweder Genese oder Dermatosen
      - Psoriasis und Parapsoriasis-Formenkreis
      - Pityriasis rubra pilaris
      - Erythematöse Erkrankungen, ausgelöst durch unterschiedlichen Noxen, z.B. Strahlen, Chemikalien, Verbrennungen etc.
      - Bullöse Dermatosen wie z.B. autoimmuner Pemphigus vulgaris, bullöses Pemphigoid
      - Erkrankungen des lichenoiden Formenkreises,
      - Pruritus (z. B. allergischer Genese)
      - Seborrhoisches Ekzem
      - Rosacea-Formenkreis
      - Erythema exsudativum multiforme
      - Balanitis
      - Vulvitis
      - Manifestation von Gefäßerkrankungen
      - Haarausfall wie Alopecia areata
      - Cutane Lymphome
   (vi) Nierenerkrankungen, die mit entzündlichen, allergischen und / oder proliferativen Prozessen einhergehen:
      - Nephrotisches Syndrom
      - Alle Nephritiden, z.B. Glomerulonephritis
   (vii) Lebererkrankungen, die mit entzündlichen, allergischen und / oder proliferativen Prozessen einhergehen:
      - akuter Leberzellzerfall
      - akute Hepatitis unterschiedlicher Genese, z.B. viral, toxisch, arzneimittelinduziert
      - chronisch aggressive und / oder chronisch intermittierende Hepatitis
   (viii) Gastrointestinale Erkrankungen, die mit entzündlichen, allergischen und / oder proliferativen Prozessen einhergehen:
      - regionale Enteritis (Morbus Crohn)
      - Colitis Ulcerosa
      - Gastritis
      - Refluxoesophagitis
      - Gastroenteritiden anderer Genese, z.B. einheimische Sprue
   (ix) Proktologische Erkrankungen, die mit entzündlichen, allergischen und / oder proliferativen Prozessen einhergehen:
      - Analekzem
      - Fissuren
      - Hämorrhoiden
      - idiopathische Proktitis
   (x) Augenerkrankungen, die mit entzündlichen, allergischen und / oder proliferativen Prozessen einhergehen:
      - allergische Keratitis, Uveitis, Iritis,
      - Konjunktivitis
      - Blepharitis
      - Neuritis nervi optici
      - Chorioditis
      - Ophtalmia sympathica
   (xi) Erkrankungen des Hals-Nasen-Ohren-Bereiches, die mit entzündlichen, allergischen und / oder proliferativen Prozessen einhergehen:
      - allergische Rhinitis, Heuschnupfen
      - Otitis externa, z.B. bedingt durch Kontaktekzem, Infektion etc.
      - Otitis media
   (xii) Neurologische Erkrankungen, die mit entzündlichen, allergischen und / oder proliferativen Prozessen einhergehen:
      - Himödem, vor allem Tumor-bedingtes Himödem
      - Multiple Sklerose
      - akute Encephalomyelitis
      - Meningitis
      - verschieden Formen von Krampfanfällen, z.B. BNS-Krämpfe
      - Akute Rückenmarksverletzung
      - Schlaganfall
   (xiii) Bluterkrankungen, die mit entzündlichen, allergischen und / oder proliferativen Prozessen einhergehen wie z.B.: M. Hodgkin oder Non-Hodgkin Lymphome, Thrombozytaemien, Erythrozytosen
      - Erworbene hämolytische Anämie
      - Idopathische Thrombocytopenia
   (xiv) Tumorerkrankungen, die mit entzündlichen, allergischen und / oder proliferativen Prozessen einhergehen wie z.B.: Karzinome oder Sarkome
      - Akute lymphatische Leukämie
      - Maligne Lymphome
      - Lymphogranulomatosen
      - Lymphosarkome
      - Ausgedehnte Metastasierungen, vor allem bei Mamma- Bronchial- und Prostatakarzinom
   (xv) Endokrine Erkrankungen, die mit entzündlichen, allergischen und / oder proliferativen Prozessen einhergehen wie z.B.:
      - Endokrine Orbitopathie
      - Thyreotoxische Krise
      - Thyreoiditis de Quervain
      - Hashimoto Thyreoiditis
      - Morbus Basedow
      - Granulomatous thyroiditis
      - Struma lymphomatosa
   (xvi) Organ- und Gewebstransplantationen , Graft-versus-host-disease(xvii) Schwere Schockzustände, z.B anaphylaktischer Schock , systemic inflammatory response syndrome (SIRS)
   (xviii)Substitutionstherapie bei:
      - angeborene primäre Nebenniereninsuffizienz, z.B. kongenitales adrenogenitales Syndrom
      - erworbene primäre Nebenniereninsuffizienz, z.B. Morbus Addison, autoimmune Adrenalitits, postinfektiös, Tumoren, Metastasen etc.
      - angeboren sekundäre Nebeniereninsuffizienz, z.B. kongenitaler Hypopitutitarismus
      - erworbene sekundäre Nebenniereninsuffizienz, z.B. postinfektiös, Tumoren etc.
   (xix) Emesis, die mit entzündlichen, allergischen und / oder proliferativen Prozessen einhergehen:
      - z.B. in Kombination mit einem 5-HT3-Antagonisten bei Zytostika - bedingten Erbrechen.
   (xx) Schmerzen bei entzündlicher Genese, z.B. Lumbago
   (xxi) Andere unterschiedliche Krankheitsstadien einschließlich Diabetes Typ I (Insulinabhängiger Diabetes), Osteoarthritis, Guillain-Barre Syndrom, Restenose nach perkutaner tramliminaler Angioplastie, Morbus Alzheimer, akute und chronische Schmerzen, Artheriosklerose, Reperfusionsverletzungen, congestive heart failure, Myokardinfarkt, thermal injury, multiple organ injury secondary to trauma, acute purulent meningitis, necrotizing enterocolitis and syndromes associated with hemodialysis, leukopheresis, and granulocyte transfusion.

Bevorzugt ist die lokale Applikation der erfindungsgemäßen Verbindungen oder deren Gemisch zur Behandlung von Erkrankungen, die unter den Punkten (i), (ii), (iii), (v), (viii). (ix), (x), (xi), (xv), (xx), (xxi) aufgeführt sind.

Die Erfindung betrifft ferner kombinierte Zusammensetzungen, worin ein Glukokortikoidrezeptor (GR) Agonist der Formel (I), oder ein pharmazeutisch akzeptables Salz davon, oder eine pharmazeutische Zusammensetzung enthaltend einen GR Agonisten der Formel (I) oder ein pharmazeutisch akzeptables Salz davon, verabreicht wird entweder gleichzeitig (gegebenenfalls in derselben Zusammensetzung) oder nacheinander zusammen mit einem oder mehreren Arzneimitteln zur Behandlung von einem der oben angesprochenen Krankheitszustände. Beispielsweise kann zur Behandlung von Rheumatoider Arthritis, Osteoarthritis, COPD (chronische obstuktive Lungenerkrankung), Asthma oder allergischer Rhinitis, ein GR Agonist der vorliegenden Erfindung kombiniert werden mit einem oder mehreren Arzneimitteln zur Behandlung von einem solchen Zustand. Wo eine solche Kombination eines GR Agonisten der Formel (I) oder eines pharmazeutisch akzeptablen Salzes davon durch Inhalation verabreicht wird, kann das zu kombinierende Arzneimittel von der folgenden Liste ausgewählt werden:
- ein PDE4 Inhibitor einschließlich einem Inhibitor der Isoform PDE4D;
- ein selektiver β.sub2. Adrenozeptor Agonist wie beispielsweise Metaproterenol, Isoproterenol, Isoprenalin, Albuterol, Salbutamol, Formoterol, Salmeterol, Terbutalin, Orciprenaline, Bitolterolmesylat, Pirbuterol oder Indacaterol;
- ein Muscarin Receptor Antagonist (zum Beispiel ein M1, M2 or M3 Antagonist, wie beispielsweise ein selektiver M3 Antagonist) wie beispielsweise Ipratropiumbromid, Tiotropiumbromid, Oxitropiumbromid, Pirenzepin oder Telenzepin;
- ein Modulator der Chemokin Rezeptor Funktion (wie beispielsweise ein CCR1 Rezeptor Antagonist); oder,
- ein Inhibitor der p38 Kinase Funktion.

Darüberhinaus können die erfindungsgemäßen Verbindungen der allgemeinen Formel (IIa) und (IIb) zur Therapie und Prophylaxe weiterer oben nicht genannter Krankheitszustände eingesetzt werden, für die heute synthetische Glucocorticoide verwendet werden (siehe dazu Hatz, HJ, Glucocorticoide: Immunologische Grundlagen, Pharmakologie und Therapierichtlinien, Wissenschafliche Verlagsgesellschaft mbH, Stuttgart, 1998).

Alle zuvor genannten Indikationen (i) bis (xx) sind ausführlich beschrieben in Hatz, HJ, Glucocorticoide: Immunologische Grundlagen, Pharmakologie und Therapierichtlinien, Wissenschafliche Verlagsgesellschaft mbH, Stuttgart, 1998.

Für die therapeutischen Wirkungen bei den oben genannten Krankheitszuständen ist die geeignete Dosis unterschiedlich und hängt beispielsweise von der Wirkstärke der Verbindung der allgemeinen Formel (IIa) und (IIb), dem Wirt, der Art der Verabreichung und der Art und der Schwere der zu behandelnden Zustände, sowie der Verwendung als Prophylaktikum oder Therapeutikum ab.

Die Erfindung betrifft die Verwendung der erfindungsgemäßen Verbindungen zur Herstellung eines Arzneimittels.

Die Erfindung liefert weiterhin
(i) die Verwendung eines der erfindungsgemäßen Verbindung gemäß Formel (IIa) und (IIb) oder deren Gemisch zur Herstellung eines Medikaments zur Behandlung von einer ERKRANKUNG;
(ii) ein Verfahren und eine Methode zur Herstellung eines Arzneimittels zur Behandlung von einer ERKRANKUNG, welches Verfahren eine Verabreichung einer Verbindungsmenge gemäß der Erfindung umfaßt, wobei die Menge die Krankheit unterdrückt, und wobei die Verbindungsmenge einem Patienten gegeben wird, der ein solches Medikament benötigt;
(iii) eine pharmazeutische Zusammensetzung zur Behandlung von einer ERKRANKUNG, welche Behandlung eines der erfindungsgemäßen Verbindungen oder deren Gemisch und wenigstens einen pharmazeutischen Hilfs- und/oder Trägerstoff umfaßt.

Insbesondere ist die Verwendung der erfindungsgemäßen Verbindungen zur Herstellung eines Arzneimittels zur Behandlng von entzündlichen Erkrankungen ein Gegenstand der Erfindung.

Im allgemeinen sind bei Tieren zufriedenstellende Resultate zu erwarten, wenn die täglichen Dosen einen Bereich von 1 µg bis 100.000 µg der erfindungsgemäßen Verbindung pro kg Körpergewicht umfassen. Bei größeren Säugetieren, beispielsweise dem Menschen, liegt eine empfohlene tägliche Dosis im Bereich von 1 µg bis 100.000 µg pro kg Körpergewicht. Bevorzugt ist eine Dosis von 10 bis 30.000 µg pro kg Körpergewicht, mehr bevorzugt eine Dosis von 10 bis 10.000 µg pro kg Körpergewicht. Zum Beispiel wird diese Dosis zweckmäßigerweise mehrmals täglich verabreicht.

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussem, Bindemitteln, Feuchthaltemitteln, Gleitmitteln, Absorptionsmitteln, Verdünnungsmitteln, Geschmackskorrigentien, Färbemitteln usw., verarbeitet und in die gewünschte Applikationsform überführt. Dabei ist auf Remington's Pharmaceutical Science, 15th ed. Mack Publishing Company, East Pennsylvania (1980) hinzuweisen. Besonders bevorzugt sind Zusätze, die für die lokale Applikation geeignet sind.

Für die orale Applikation kommen insbesondere Tabletten, Dragees, Kapseln, Pillen, Pulver, Granulate, Pastillen, Suspensionen, Emulsionen oder Lösungen in Frage.

Für die parenterale Applikation sind Injektionszubereitungen möglich.

Für die intraartikulären Injektion können entsprechend zubereitet Kristallsuspensionen verwendet werden.

Für die intramuskuläre Injektion können wässrige und ölige Injektionslösungen oder Suspensionen und entprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen, als auch zur lokalen Therapie verwendet werden.

Zur pulmonalen Applikation der neuen Verbindungen können diese in Form von Aerosolen und Inhalaten verwendet werden.

Für die lokale Anwendung an Augen, äußerem Gehörgang, Mittelohr, Nasenhöhle und Nasennebenhöhlen können die neuen Verbindungen als Tropfen, Salben und Tinkturen in entsprechenden pharmazeutischen Zubereitungen verwendet werden.

Für die topische Auftragung sind Formulierungen in Gelen, Salben, Fettsalben, Cremes, Pasten, Puder, Emulsion, Lösung und Suspensionen möglich. Die Dosierung der Verbindungen der allgemeinen Formel (IIa) und (IIb) sollte in diesen Zubereitungen 0.01% - 20% betragen, um eine ausreichende pharmakologische Wirkung zu erzielen.

Die Erfindung umfaßt ebenfalls die erfindungsgemäßen Verbindungen der allgemeinen Formel (IIa) und (IIb) als therapeutischen Wirkstoff. Weiterhin gehört zur Erfindung die erfindungsgemäßen Verbindungen der allgemeinen Formel (IIa) und (IIb) als therapeutischen Wirkstoff zusammen mit pharmazeutisch verträglichen und annehmbaren Hilfsstoffen und Trägerstoffen. Ebenfalls umfaßt die Erfindung eine pharmazeutische Zusammensetzung, die eine der pharmazeutisch aktiven, erfindungsgemäßen Verbindungen oder deren Gemisch oder deren pharamzeutisch verträgliches Salz und ein pharmazeutisch verträgliches Salz oder pharmazeutisch verträgliche Hilfsstoffe und Trägerstoffe enthält.

Die nachstehenden Beispiele dienen der näheren Erläuterung der Erfindung ohne sie darauf beschränken zu wollen. Die Synthesen von wichtigen Vorstufen, die im Rahmen des experimentellen Teils nicht offenbart sind, sind bereits Stand der Technik, und können zum Beispiel aus der WO 98/54159 und WO 02110143, WO03/082280 oder WO03/082827 entnommen werden.

### Experimenteller Teil

### Beispiel 1

### 5-[4-(5-Fluor-2,3-dihydrobenzofuran-7-yl)-2-hydroxy-4-methyl-2-trifluormethyl-pentylamino]-2-methylchinolin

### 4-(5-Fluor-2-hydroxyphenyl)-2-hydroxy-4-methyl-2-trifluormethyl-valeriansäure-ethylester

Eine Lösung von 4-(5-Fluor-2-methoxyphenyl)-2-hydroxy-4-methyl-2-trifluormethyl-valeriansäure-ethylester (WO 00/32584) (10.83 g, 30.74 mmol) in Dichlormethan (200 mL) wird unter Eisbadkühlung mit 1 M Bortribromid-Chloroform-Lösung (60 mL) versetzt und 3 h bei 2 - 4 °C gerührt. Der Ansatz auf Eis und ges. NaHCO₃-Lösung gegossen und 30 min unter Eiskühlung gerührt. Die organische Phase wird abgetrennt und die wäßrige Phase noch zweimal mit Dichlormethan extrahiert. Die vereinigten organischen Extrakte werden mit ges. NaCl-Lösung gewaschen, getrocknet (Na₂SO₄) und i. Vak. eingeengt. Säulenchromatographie des Rückstands (Kieselgel) mit Hexan-Essigester liefert 5.36 g Produkt. Extraktion der wäßrigen Phase mit Essigester liefert nochmals 4.0 g Produkt.
¹H-NMR (300 MHz, CDCl₃); δ = 1.22 (t, 3H), 1.41 (s, 3H), 1.47 (s, 3H), 2.52 (d, 1 H), 2.87 (d, 1H), 3.55 (br., 1H), 3.76 (dq, 1 H), 4.11 (dq, 1H), 5.01 (s, 1H), 6.59 (dd, 1H), 6.77 (ddd, 1 H), 6.90 (dd, 1 H).

### 4-(2-Allyloxy-5-fluorphenyl)-2-hydroxy-4-methyl-2-trifluormethyl-valeriansäure-ethylester

Zu einer Lösung von 4-(5-Fluor-2-hydroxyphenyl)-2-hydroxy-4-methyl-2-trifluormethyl-valeriansäure-ethylester (5.36 g, 15.84 mmol) in DMF (50 mL) werden unter Eiskühlung Kaliumcarbonat (4.15 g, 30 mmol) und Allylbromid (2.16 mL, 25 mmol) gegeben. Nach 2 h bei 2 °C und 2 h bei Raumtemperatur wird der Ansatz in Eiswasser gegossen und mit Hexan-Ether 2:1 extrahiert. Die vereinigten organischen Extrakte werden getrocknet (Na₂SO₄) und i. Vak. eingeengt. Säulenchromatographie an Kieselgel mit Hexan-Essigester liefert 5.7 g des Produkts.
¹H-NMR (300 MHz, CDCl₃); δ = 1.18 (t, 3H), 1.39 (s, 3H), 1.45 (s, 3H), 2.54 (d, 1H), 2.91 (d, 1H), 3.48 (br., 1H), 3.65 (dq, 1H), 4.09 (dq, 1H), 4.55 (dt, 2H), 5.31 (dq, 1H), 5.45 (dq, 1H), 6.09 (ddt, 1H), 6.76 (dd, 1H), 6.84 (ddd, 1H), 6.91 (dd, 1 H).

### 4-(3-Allyl-5-fluor-2-hydroxyphenyl)-2-hydroxy-4-methyl-2-trifluormethyl-valeriansäure-ethylester

4-(2-Allyloxy-5-fluorphenyl)-2-hydroxy-4-methyl-2-trifluormethyl-valeriansäure-ethylester (5.65 g, 14.93 mmol) wird in der Mikrowelle 10 min auf 230 °C erhitzt. Das Reaktionsgemisch wird säulenchromatographisch an Kieselgel mit Hexan-Essigester gereinigt. 3.31 g des Produkts werden erhalten.
¹H-NMR (300 MHz, CDCl₃); δ = 1.23 (t, 3H), 1.40 (s, 3H), 1.45 (s, 3H), 2.60 (d, 1H), 2.78 (d, 1H), 3.37 (d, 2H), 3.49 (br., 1 H), 3.83 (dq, 1H), 4.14 (dq, 1H), 5.09 (br., 1H), 5.23 (dq, 1H), 5.26 (dq, 1H), 5.99 (ddt, 1H), 6.72 (dd, 1 H), 6.83 (dd, 1 H).

### 4-(5-Fluor-2-hydroxy-3-(2-hydroxyethyl)phenyl)-2-hydroxy-4-methyl-2-trifluormethyl-valeriansäure-ethylester

4-(3-Allyl-5-fluor-2-hydroxyphenyl)-2-hydroxy-4-methyl-2-trifluormethyl-valeriansäure-ethylester (4.9 g, 12.95 mmol) in Aceton (214 mL) und Wasser (32 mL) werden unter Eiskühlung mit N-Methylmorpholinoxid-Hydrat (1.75 g, 12.95 mmol) und 0.4 mL Osmiumtetroxid-Lösung (2.5 Gew.-Proz. in *tert-*Butanol) versetzt. Nach 30 min bei 2 °C und 16 h bei Raumtemperatur wird der Ansatz mit weiteren 0.3 mL Osmiumtetroxid-Lösung versetzt und 3 Tage bei Raumtemperatur gerührt. Zur Aufarbeitung wird Aceton im Rotationsverdampfer abdestilliert, der Rückstand in Essigester (200 mL) und Wasser (150 mL) aufgenommen und die Phasen getrennt. Die wäßrige Phase wird noch zweimal mit Essigester extrahiert, die vereinigten Essigester-Extrakte mit ges. NaCl-Lösung gewaschen, getrocknet (Na₂SO₄) und eingeengt. Säulenchromatographie an Kieselgel mit Hexan-Essigester liefert 5.27 g 4-(3-(2,3-Dihydroxypropyl)-5-fluor-2-hydroxyphenyl)-2-hydroxy-4-methyl-2-trifluormethyl-valeriansäure-ethylester. Dieser (5.2 g, 12.6 mmol) wird mit Natriumperiodat (5.39, 25.2 mmol) in THF (75 mL) und Wasser (12.5 mL) 24 h unter Stickstoff gerührt. Der Ansatz wird eingeengt und der wäßrige Rückstand dreimal mit Essigester extrahiert. Die vereinigten organischen Phasen werden mit ges. NaCl-Lösung gewaschen, getrocknet (Na₂SO₄) und i. Vak. eingeengt. Säulenchromatographie an Kieselgel mit Hexan-Essigester liefert 4.3 g 4-(5-Fluor-2-hydroxy-2,3-dihydrobenzofuran-7-yl)-2-hydroxy-4-methyl-2-trifluormethyl-valeriansäure-ethylester. Davon werden 4.1 g (10.78 mmol) in Methanol (150 mL) gelöst, die Lösung portionsweise mit Natriumborhydrid (586 mg, 15 mmol) versetzt und 30 min bei Raumtemperatur gerührt. Mit Essigsäure wird ein pH-Wert von 7.5 eingestellt und das Reaktionsgemisch eingeengt. Der Rückstand wird in Essigester (200 mL) und ges. NaHCO₃-Lösun (75 mL) aufgenommen, die Phasen getrennt, die organische Phase mit ges. NaCl-Lösung gewaschen, getrocknet (Na₂SO₄) und i. Vak. eingeengt, wobei 4.01 g Produkt anfallen.
¹H-NMR (300 MHz, CDCl₃); δ = 1.23 (t, 3H), 1.40 (s, 3H), 1.47 (s, 3H), 2.59 (d, 1 H), 2.76-2.92 (m, 2H), 2.91 (d, 1H), 3.85 (dq, 1H), 3.98 (m, 2H), 4.05 (dq, 1H), 6.67 (dd, 1H), 6.81 (dd, 1H).

### 4-(5-Fluor-2,3-dihydrobenzofuran-7-yl)-2-hydroxy-4-methyl-2-trifluormethyl-valeriansäure-ethylester

4-(5-Fluor-2-hydroxy-3-(2-hydroxyethyl)phenyl)-2-hydroxy-4-methyl-2-trifluormethyl-valeriansäure-ethylester (3.90 g, 10.2 mmol) wird mit Triphenyphosphin (3.14 g, 12 mmol) und Triethylamin (2.1 mL, 15 mmol) in Acetonitril (150 mL) gelöst, mit Tetrachlorkohlenstoff (2 mL) versetzt und 3 Tage in einer Stickstoffatmosphäre bei Raumtemperatur gerührt. Das Lösungsmittel wird im Rotationsverdampfer abdestilliert und der Rückstand in Essigester (150 mL) und Wasser (75 mL) aufgenommen. Die Essigester-Phase wird abgetrennt, mit ges. NaCl-Lösung gewaschen, getrocknet (Na₂SO₄) und i. Vak. eingeengt. Säulenchromatographie an Kieselgel mit Hexan-Essigester liefert 3.31 g des Produkts.
¹H-NMR (300 MHz, CDCl₃); δ = 1.21 (t, 3H), 1.35 (s, 3H), 1.40 (s, 3H), 2.43 (d, 1 H), 2.74 (d, 1H), 3.15 (m, 2H), 3.56 (br., 1H), 3.73 (dq, 1H), 4.13 (dq, 1H), 4.58 (t, 2H), 6.68 (dd, 1H), 6.77 (dm, 1 H).

### 4-(5-Fluor-2,3-dihydrobenzofuran-7-yl)-4-methyl-2-trifluormethyl-1,2-pentadiol

Eine Lösung von 4-(5-Fluor-2,3-dihydrobenzofuran-7-yl)-2-hydroxy-4-methyl-2-trifluormethyl-valeriansäure-ethylester (3.2 g, 8.78 mmol) in Ether (150 mL) wird unter Eiskühlung mit Lithiumaluminiumhydrid (683 mg, 18 mmol) versetzt und 1 h bei 2 °C und 6 h bei Raumtemperatur gerührt. Der Ansatz wird auf 3 °C gekühlt, ges. HaHCO₃-Lösung (1.5 mL) dazugetropft, 30 min bei 3 °C und 16 h bei Raumtamp. gerührt. Der farblose Niederschlag wird abgesaugt und mit Ether gewaschen. Die vereinigten Filtrate werden eingeengt und säulenchromatographisch an Kieselgel mit Hexan-Essigester gereinigt. 2.65 g des Produkts fallen als farbloser, kristalliner Feststoff an.
¹H-NMR (300 MHz, CDCl₃); δ = 1.39 (s, 3H), 1.47 (s, 3H), 2.21 (d, 1H), 2.46 (d, 1 H), 2.89 (br., 1H), 3.17 (t, 2H), 3.41 (dm, 1H), 3.49 (d, 1H), 4.57 (t, 2H), 6.80 (d, 2H).

### 4-(5-Fluor-2,3-dihydrobenzofuran-7-yl)-2-hydroxy-4-methyl-2-trifluormethylpentanal

Zu einer Lösung von 4-(5-Fluor-2,3-dihydrobenzofuran-7-yl)-4-methyl-2-trifluormethyl-1,2-pentadiol (2.61 g, 8.1 mmol), Dimethylsulfoxid (28.6 mL) und Triethylamin (5.6 mL, 40 mmol) in Dichlormethan (85 mL) wird unter Stickstoffatmosphäre Pyridin-Schwefeltrioxid-Komplex (3.82 g, 24 mmol) gegeben. Der Ansatz wird 3 h bei Raumtemp. gerührt, mit ges. NH₄Cl-Lösung (50 mL) versetzt, 30 min bei Raumtemp. gerührt und mit Ether (250 mL) verdünnt. Die Phasen werden getrennt und die wäßrige Phase mit Ether extrahiert. Die vereinigten organischen Phasen werden mit ges. NaCl-Lösung gewaschen, getrocknet (Na₂SO₄) und i. Vak. eingeengt. Säulenchromatographie des Rückstands an Kieselgel mit Hexan-Essigester liefert 2.19 g des Produkts.
¹H-NMR (300 MHz, CDCl₃); δ = 1.35 (s, 3H), 1.42 (s, 3H), 2.20 (d, 1H), 3.17 (t, 2H), 3.28 (d, 1H), 3.62 (s, 1H), 4.59 (m, 2H), 6.63 (dd, 1H), 6.81 (dm, 1H), 9.08 (s, 1H).

### 2-Methyl-5-nitrochinolin

Zu 65 proz. Salpetersäure (61 mL, 0.88 mol) wird bei einer Innentemperatur von 0 - 10 °C (Trockeneiskühlung) innerhalb von 45 min 2-Methylchinolin (108.3 mL, 0.80 mol) getropft. Nach 1 h wird das ausgefallene Nitrat abgesaugt und portionsweise bei einer Innentemperatur von 0 - 6 °C in konz. Schwefelsäure (240 mL) eingetragen. Nach 30 min wird Kaliumnitrat (6 g, 60 mmol) dazugegeben und 16 h bei Raumtemperatur gerührt. Der Ansatz wird langsam auf Eis/ Wasser gegossen und mit 40 proz. NaOH (~ 500 mL) ein pH-Wert von 1.5 eingestellt. Der Niederschlag wird abgesaugt, das Filtrat mit 25 proz. Ammoniakwasser alkalisch (pH 10) gestellt und filtriert. Der Filterrückstand wird in heißem Methanol (500 mL) gelöst. Beim Abkühlen kristallisiert sich das 8-Nitro-Isomer aus. Die Mutterlauge wird eingeengt und säulenchromatographisch an Kieselgel mit Hexan-Essigester gereinigt, wobei 53 g des 2-Methyl-5-nitrochinolins anfallen.
¹H-NMR (300 MHz, CDCl); δ= 2.79 (s, 3H), 7.52 (d, 1 H), 7.76 (d, 1 H), 8.31 (d, 1 H), 8.32 (d, 1 H), 8.88 (d, 1 H).

### 5-Amino-2-methylchinolin

2-Methyl-5-nitrochinolin (25 g, 132.85 mmol) und Palladium auf Kohlenstoff (2.5 g) in 8 mL Eisessig werden 5¹/₂ h in einer Wasserstoffatmosphäre bei Normaldruck gerührt. Der Katalysator wird abgesaugt und mit Essigester gewaschen. Die vereinigten Filtrate werden eingeengt. Säulenchromatographie des Rückstands an Kieselgel mit Dichlormethan-Aceton liefert 10.6 g des Produkts.
¹H-NMR (300 MHz, CDCl₃; δ= 2.72 (s, 3H), 4.15 (br., 2H), 6.76 (dd, 1H), 7.23 (d, 1 H), 7.43-7.50 (m, 2H), 8.06 (d, 1H).

### 5-[4-(5-Fluor-2,3-dihydrobenzofuran-7-yl)-2-hydroxy-4-methyl-2-trifluormethylpentylidenamino]-2-methylchinolin

Eine Mischung aus 4-(5-Fluor-2,3-dihydrobenzofuran-7-yl)-2-hydroxy-4-methyl-2-trifluormethyl-pentanal (320 mg, 1 mmol) und 5-Amino-2-methylchinolin (190 mg, 1.2 mmol) in Essigsäure (2 mL) wird 16 h bei Raumtemperatur gerührt., mit 10 mL Toluol verdünnt und 4 h am Wasserabscheider erhitzt. Der Ansatz wird eingeengt, wobei die Essigsäure azeotrop mit Toluol entfernt wird. Der Rückstand wird säulenchromatographisch an Kieselgel mit Hexan-Essigester gereinigt: 274 mg des Produkts fallen als farblose Kristalle an.
¹H-NMR (300 MHz, CDCl₃); δ = 1.34 (s, 3H), 1.54 (s, 3H), 2.27 (d, 1 H), 2.66 (m, 1 H), 2.76 (s, 3H), 2.94 (m, 1 H), 3.29 (d, 1 H), 4.47 (m, 2H), 4.85 (s, 1 H), 6.28 (dm, 1 H), 6.51 (d, 1 H), 6.61 (dd, 1 H), 7.33 (d, 1 H), 7.51 (t, 1 H), 7.63 (s, 1 H), 7.90 (d, 1 H), 8.18 (d, 1 H).

### 5-[4-(5-Fluor-2,3-dihydrobenzofuran-7-yl)-2-hydroxy-4-methyl-2-trifluormethylpentylamino]-2-methylchinolin

5-[4-(5-Fluor-2,3-dihydrobenzofuran-7-yl)-2-hydroxy-4-methyl-2-trifluormethylpentylidenamino]-2-methylchinolin (266 mg, 0.58 mmol) und Natriumhydrogencarbonat (250 mg) in Methanol (15 mL) werden 15 min bei Raumtemperatur. gerührt, Natriumborhydrid (152 mg, 4 mmol) wird in 4 Portionen über 24 h dazugegeben. Nach Beendigung der Reaktion (DC-Kontrolle) wird der Ansatz mit ges, NaHCO₃-Lösung (10 mL) versetzt und eingeengt. Der Rückstand wird in Essigester (30 mL) und Wasser (20 mL) aufgenommen und die Phasen getrennt. Die wäßrige Phase wird mit Essigester extrahiert. Die vereinigten organischen Phasen werden getrocknet (Na₂SO₄) und eingeengt. Säulenchromatographie an Kieslegel mit Hexan-Essigester liefert 200 mg des Produkts.
¹H-NMR (300 MHz, CDCl₃); δ = 1.43 (s, 3H), 1.54 (s, 3H), 2.29 (d, 1H), 2.68 (d, 1 H), 2.71 (s, 3H), 2.92-3.19 (m, 3H), 3.34 (dd, 1H), 4.26 (br., 1H), 4.52 (m, 2H), 6.09 (dm, 1H), 6.81 (dm, 1H), 6.87 (dm, 1H), 7.20 (d, 1H), 7.39-7.47 (m, 2H), 7.89 (d, 1H).

Die Enantiomere werden mittels chiraler HPLC unter Verwendung des Säulentyps Chiralpak AD 20 µ und dem Eluenten Hexan (0.1 % Diethyamin) - Ethanol in (+)- und (-)-Isomer getrennt. Das (-)-Enantiomer ([α]_{D}(THF) -4.3,2°, c = 1.45) wird vor dem (+)-Enantiomer (([α]_{D}(THF) +42,8°, c = 1.53) eluiert. Die R-konfigurierte Verbindung ist das Enantiomere, während die S-konfigurierte Verbindung das (+)-Enantiomere ist.

### Beispiel 2

### 5-[4-(5-Fluor-2,3-dihydrobenzofuran-7-yl)-2-hydroxy-4-methyl-2-trifluormethylpentylamino]-1-methylisochinolin

Analog zu Beispiel 1 wird 4-(5-Fluor-2,3-dihydrobenzofuran-7-yl)-2-hydroxy-4-methyl-2-trifluormethyl-pentanal mit 5-Amino-1-methylisochinolin in 5-[4-(5-Fluor-2,3-dihydrobenzofuran-7-yl)-2-hydroxy-4-methyl-2-trifluormethylpentylidenamino]-1-methylisochinolin überführt, das mit Natriumborhydrid zum Produkt reduziert wird.
¹H-NMR (300 MHz, CDCl₃); δ = 1.43 (s, 3H), 1.55 (s, 3H), 2.29 (d, 1 H), 2.69 (d, 1 H), 2.90 (s, 3H), 2.90-3.20 (m, 4H), 3.33 (br., 1 H), 4.35 (br., 1 H), 4.53 (m, 2H), 6.26 (d, 1 H), 6.80 (dm, 1 H), 6.88 (dm, 1 H), 7.29 (t, 1 H), 7.35 (d, 1 H), 7.48 (d, 2H), 8.32 (d, 1 H).
Trennung der Enantiomere mittels chiraler HPLC (Säule: Chiralpak AD 20 µ, Eluens: Hexan-Ethanol) liefert zuerst das (+)-Enantiomer ([α]_{D}(MeOH) +29.8°, c = 0.54) und dann das (-)-Enantiomer ([α]_{D}(MeOH) -29.4°, c = 0.55).

### Beispiel 3

### 5-[4-(5-Fluor-2,3-dihydrobenzofuran-7-yl)-2-hydroxy-4-methyl-2-trifluoromethylpentylamino]isochinol-1(2H)-on

Analog zu Beispiel 1 wird 4-(5-Fluor-2,3-dihydrobenzofuran-7-yl)-2-hydroxy-4-methyl-2-trifluormethyl-pentanal mit 5-Aminoisochinol-2(1 H)-on in 5-[4-(5-Fluor-2,3-dihydrobenzofuran-7-yl)-2-hydroxy-4-methyl-2-trifluormethylpentylidenamino]isochinol-1(2H)-on überführt, das mit Natriumborhydrid zum Produkt reduziert wird.
¹H-NMR (300 MHz, [D]₆-DMSO); δ = 1.33 (s, 3H), 1.52 (s, 3H), 1.98 (d, 1H), 2.78 (d, 1H), 2.84-3.10 (m, 4H), 4.49 (t, 1H), 4.80 (t, 1H), 6.03 (s, 1H), 6.21 (d, 1H), 6.41 (d, 1H), 6.80-6.87 (m, 2H), 7.12-7.17 (m, 2H), 7.47 (d, 1H), 11.21 (br. d, 1H).

Trennung der Enantiomere mittels chiraler HPLC (Säule: Chiralpak AS 20 µ, Eluens: Hexan-Ethanol) liefert zuerst das (+)-Enantiomer ([α]_{D}(MeOH) +29.9°, c = 0.92) und dann das (-)-Enantiomer ([α]_{D}(MeOH) -28.4°, c = 0.94).

### Beispiel 4

### 5-[4-(5-Fluor-2,3-dihydrobenzofuran-7-yl)-2-hydroxy-4-methyl-2-trifluormethylpentylamino]-2,6-dimethylchinolin

Analog zu Beispiel 1 wird 4-(5-Fluor-2,3-dihydrobenzofuran-7-yl)-2-hydroxy-4-methyl-2-trifluormethyl-pentanal mit 5-Amino-2,6-dimethylchinolin in 5-[4-(5-Fluor-2,3-dihydrobenzofuran-7-yl)-2-hydroxy-4-methyl-2-trifluormethylpentylidenamino]-2,6-dimethylchinolin überführt, das mit Natriumcyanoborhydrid zum Produkt reduziert wird.
¹H-NMR (300 MHz, CDCl₃); δ = 1.34 (s, 3H), 1.57 (s, 3H), 2.22 (d, 1 H), 2.31 (s, 3H), 2.45 (d, 1 H), 2.66-2.76 (m, 1 H), 2.74 (s, 3H), 2.83-3.00 (m, 2H), 3.10 (d, 1 H), 3.52 (br. 1 H), 4.20 (q, 1 H), 4.29 (s, 1 H), 4.38 (q, 1 H), 6.55 (d, 1 H), 6.77 (dm, 1 H), 7.22 (d, 1 H), 7.42 (d, 1 H), 7.68 (d, 1 H), 7.94 (d, 1 H).
Trennung der Enantiomere mittels chiraler HPLC (Säule: Chiralcel OJ 5 µ, Eluens: Hexan-Ethanol) liefert zuerst das (+)-Enantiomer ([α]_{D}(MeOH) +55.8°, c = 0.94) und dann das (-)-Enantiomer ([α]_{D}(MeOH) -52.1 °, c = 0.99).

### Beispiel 5

### 5-[4-(5-Fluor-2,3-dihydrobenzofuran-7-yl)-2-hydroxy-4-methyl-2-trifluormethylpentylamino]-6-chlor-2-methylchinolin

Analog zu Beispiel 1 wird 4-(5-Fluor-2,3-dihydrobenzofuran-7-yl)-2-hydroxy-4-methyl-2-trifluormethyl-pentanal mit 5-Amino-6-chlor-2-methylchinolin in 5-[4-(5-Fluor-2,3-dihydrobenzofuran-7-yl)-2-hydroxy-4-methyl-2-trifluormethylpentylidenamino]-6-chlor-2-methylchinolin überführt, das mit Natriumcyanoborhydrid zum Produkt reduziert wird.
¹H-NMR (300 MHz, CDCl₃); δ = 1.34 (s, 3H), 1.57 (s, 3H), 2.22 (d, 1 H), 2.53 (d, 1 H), 2.75 (s, 3H), 2.72-2.83 (m, 1 H), 2.89-3.02 (m, 2H), 3.16 (dd, 1 H), 4.04 (s, 1 H), 4.30 (q, 1 H), 4.42 (q, 1 H), 6.51 (dm, 1 H), 6.73 (dd, 1 H), 7.26 (d, 1 H), 7.55 (d, 1 H), 7.66 (d, 1 H), 7.96 (d, 1 H).

Trennung der Enantiomere mittels chiraler HPLC (Säule: Chiralcel OJ 20 µ, Eluens: Hexan-Ethanol) liefert zuerst das (+)-Enantiomer ([α]_{D}(MeOH) +41.7°, c = 0.88) und dann das (-)-Enantiomer ([α]_{D}(MeOH) -39.8°, c = 0.99).

### Beispiel 6

### 5-[4-(5-Fluor-2,3-dihydrobenzofuran-7-yl)-2-hydroxy-4-methyl-2-trifluormethylpentylamino]isochinolin

Analog zu Beispiel 1 wird 4-(5-Fluor-2,3-dihydrobenzofuran-7-yl)-2-hydroxy-4-methyl-2-trifluormethyl-pentanal mit 5-Aminoisochinolin in 5-[4-(5-Fluor-2,3-dihydrobenzofuran-7-yl)-2-hydroxy-4-methyl-2-trifluormethylpentylidenamino]isochinolin überführt, das mit Natriumborhydrid zum Produkt reduziert wird.
¹H-NMR (300 MHz, CDCl₃)-, δ = 1.43 (s, 3H), 1.55 (s, 3H), 2.30 (d, 1H), 2.71 (d, 1H), 2.92 (m, 1H), 3.07 (m, 1H), 3.17 (dd, 1H), 3.35 (dd, 1H), 4.35 (br.t, 1H), 4.49 (q, 1H), 4.55 (q, 1 H), 6.27 (m, 1H), 6.78 (dm, 1H), 6.88 (dm, 1H), 7.36 (m, 2H), 7.40 (d, 1H), 8.45 (d, 1H), 9.13 (s, 1H).

### Beispiel 7

### 5-[4-(5-Fluor-2,3-dihydrobenzofuran-7-yl)-2-hydroxy-4-methyl-2-trifluormethylpentylamino]chinolin

Analog zu Beispiel 1 wird 4-(5-Fluor-2,3-dihydrobenzofuran-7-yl)-2-hydroxy-4-methyl-2-trifluormethyl-pentanal mit 5-Aminochinolin in 5-[4-(5-Fluor-2,3-dihydrobenzofuran-7-yl)-2-hydroxy-4-methyl-2-trifluormethylpentylidenamino]chinolin überführt, das mit Natriumborhydrid zum Produkt reduziert wird.
¹H-NMR (300 MHz, CDCl₃); δ = 1.43 (s, 3H), 1.54 (s, 3H), 2.31 (d, 1H), 2.68 (d, 1 H), 2.96 (m, 1H), 3.08 (m, 1H), 3.17 (dd, 1H), 3.35 (dd, 1 H), 4.32 (br.t, 1H), 4.52 (m, 2H), 6.15 (d, 1H), 6.80 (dm, 1H), 6.88 (dd, 1H), 7.31 (dd, 1H), 7.45 (t, 1H), 7.53 (d, 1H), 7.98 (d, 1H), 8.86 (dd, 1H).

### Beispiel 8

### 5-[4-(2,3-Dihydro-5-fluoro-7-benzofuranyl)-2-hydroxy-4-methyl-2-trifluormethylpentylamino]chinolin-2[1H]-on

Analog Beispiel 1 wird ausgehend von 250 mg 4-(2,3-Dihydro-5-fluoro-7-benzofuranyl)-2-hydroxy-4-methyl-2-trifluormethyl-pentanal und 124 mg 5-Aminochinolin-2[1 H]-on das entsprechende lmin hergestellt. Nach Umsetzung mit Natriumcyanoborhydrid erhält man die Titelverbindung.
¹H-NMR (CD₃OD): δ = 1.38 (s, 3H), 1.60 (s, 3H), 2.74-2.88 (m, 1H), 2.94-3.05 (m, 4H), 3.05-3.17 (m, 1 H), 4.50 (t, 2H), 5.83 (d, 1H), 6.52 (d, 1H), 6.62-6.72 (m, 2H), 6.83 (dd, 1H), 7.22 (t, 1H), 7.94 (d, 1H)

**Beispiel 9**

### 6-Fluor-5-[4-(5-fluor-2,3-dihydrobenzofuran-7-yl)-2-hydroxy-4-methyl-2-trifluormethyl-pentylamino]-2-methylchinolin

Analog Beispiel 1 wird ausgehend von 250 mg 4-(2,3-Dihydro-5-fluoro-7-benzofuranyl)-2-hydroxy-4-methyl-2-trifluormethyl-pentanal und 138 mg 5-Amino-6-fluor-2-methylchinolin das entsprechende Imin hergestellt. Nach Umsetzung mit Natriumcyanoborhydrid erhält man die Titelverbindung.
¹H-NMR (CD₃OD): δ = 1.36 (s, 3H), 1.57 (s, 3H), 2.01 (d, 1H), 2.72 (s, 3H), 2.74-2.84 (m, 1 H), 2.92 (d, 1 H), 2.94-3.08 (m, 1 H), 3.23 (d, 1 H), 3.31 (d, 1 H), 4.34-4.53 (m, 2H), 6.62 (d, 1 H), 6.75 (dd, 1 H), 7.34-7.49 (m, 3H), 8.19 (d, 1 H)

### Beispiel 10

### 8-Fluor-5-[4-(5-Fluor-2,3-dihydrobenzofuran-7-yl)-2-hydroxy-4-methyl-2-trifluormethyl-pentylamino]-2-methylchinolin

Analog Beispiel 1 wird ausgehend von 45 mg 4-(2,3-Dihydro-5-fluoro-7-benzofuranyl)-2-hydroxy-4-methyl-2-trifluormethyl-pentanal und 25 mg 5-Amino-8-fluor-2-methylchinolin das entsprechende Imin hergestellt. Nach Umsetzung mit Natriumcyanoborhydrid erhält man die Titelverbindung.
¹H-NMR (CD₃OD): δ = 1.38 (s, 3H), 1.62 (s, 3H), 2.01 (d, 1 H), 2.53 (dt, 1 H), 2.73 (s, 3H), 2.84-3.22 (m, 4H), 4.44 (dt, 2H), 5.90 (dd, 1 H), 6.66 (dd, 1 H), 6.82 (dd, 1 H), 7.14 (dd, 1 H), 7.40 (d, 1 H), 8.21 (dd, 1 H).

### Beispiel 11

### 5-[4-(5-Fluor-2,3-dihydrobenzofuran-7-yl)-2-hydroxy-4-methyl-2-trifluormethylpentylamino]-2-methylisochinol-1(2H)-on

Analog zu Beispiel 1 wird 4-(2,3-Dihydro-5-fluoro-7-benzofuranyl)-2-hydroxy-4-methyl-2-trifluormethyl-pentanal mit 5-Amino-2-methylisochinol-1(2H)-on ins entsprechende lmin überführt. Nach Umsetzung mit Natriumborhydrid erhält man die Titelverbindung.
¹H-NMR (CDCl₃): δ = 1.40 (s, 3H), 1.55 (s, 3H), 2.25 (d, 1H), 2.65 (d, 1H), 2.95-3.30 (m, 4H), 3.60 (s, 3H), 4.00 (br., 1H), 4.50 (q, 1 H), 4.55 (q, 1H), 6.25 (d, 1H), 6.30 (d, 1H), 6.80 (dm, 1H), 6.90 (dm, 1H), 7.05 (d, 1H), 7.25 (t, 1H), 7.85 (d, 1H).

Die Trennung in die Enantiomeren erfolgte an einer chiralen Säule (Chiralpak AD-H 5µ, Eluenten Hexan/Ethanol), die Drehwerte für die Enantiomeren betragen:
[α]_{D}=+31,5±0,2 (c= 1 Methanol) und [α]D= -32,4±0,1 (c = 0,99 Methanol)

### Beispiel 12

### 5-[4-(5-Fluor-2,3-dihydrobenzofuran-7-yl)-2-hydroxy-4-methyl-2-trifluormethylpentylamino]-2-methylchinolin

### a) 2-Hydroxy-4-methyl-2-trifluormethyl-pent-4-ensäureethylester

3.2g Eisen(III)chlorid werden in 1000ml Dichlormethan suspendiert und auf - 10°C gekühlt. 100g Trifluorethylpyruvat werden zugegeben und eine Stunde gerührt. Ca. 140g Isobuten werden unter Kühlung auf ca. -50°C einkondensiert und die Mischung fünf Stunden bei -40°C bis -50°C gerührt, sowie über Nacht nachgerührt. Nach üblicher wässriger Aufarbeitung werden die wässrigen Phasen vereinigt und mit Dichlormethan gewaschen, die organischen Phasen vereinigt, mit Aktivkohle versetzt, 30min gerührt, filtriert und eingeengt. Zur weiteren Reinigung wird das Produkt in einem Cyclohexan (100 ml) / Methanol (120 ml) Gemisch aufgenommen und die Phasen werden getrennt. Die produkthaltige Methanolphase wird eingeengt.
Ausbeute: 118,0g = 89% d.Th.
¹H-NMR (600 MHz, CDCl₃); δ =1.35 (t, 3H), 1.79 (s, 3H), 2.59 (d, 1 H), 2.76 (d, 1 H), 3.87 (s, 1 H), 4.325 (dq, 1 H), 4.365 (dq, 1 H), 4.82 (s, 1 H), 4.92 (s, 1 H).

### Enantiomerentrennung:

200mg des in 12a) erhaltenen Esters werden in 2ml Hexan gelöst und auf der 5cm Prochromanlage (Chiralpack AD) bei einem Gegendruck von 2bar getrennt; Eluenz: Hexan/0,1 % Trifluoressigsäure. Es werden zwei Fraktionen erhalten. Enantiomer I (eluiert bei den angegebenen HPLC-Methoden zuerst; Chiralpak AS250-0,46mm: 7,58 min / Chiralpak AD-H-5µ: 6,8 min):
[α]_{D} = -6,1° +/- 0,2° (c = 0,944; CHCl₃)

Enantiomer II (eluiert bei den angegebenen HPLC-Methoden als 2. Verbindung; Chiralpak AS250-4,6µ: 9,17 min / Chiralpak AD-H-5µ: 8,2 min):
[α]_{D} = +5,9° +/- 0,5° ( c = 1,072; CHCl₃)

### b) 4-(5-Fluor-2,3-dihydrobenzofuran-7-yl)-2-hydroxy-4-methyl-2-trifluormethyl-valeriansäure-ethylester

18,32g 5-Fluor-2,3-dihydrobenzofuran werden vorgelegt und auf 0°C gekühlt. Unter starkem Rühren werden 11,77g Aluminium(III)chlorid in einer Portion zugegeben. Die Temperatur wird gehalten und nun 10,00g der in Beispiel 12a) hergestellten Verbindung langsam zugetropft. Man lässt den Ansatz auf Raumtemperatur kommen und rührt ca. 7h nach. Es werden je 50ml Ethylacetat und Wasser zugegeben und 15min gerührt. Nach Zugabe von 5ml konzentrierter Salzsäure werden die Phasen getrennt und die organische Phase mit Natriumhydrogencarbonatlösung ausgerührt. Die organische Phase wird mit Wasser und gesättigter Natriumchloridlösung gewaschen und eingeengt. Nach Kugelrohrdestillation bei 85°C / 1 mbar und abschließender Kristallisation des Rohproduktes (Sumpf) aus Ethanol (100 ml) / Wasser (80 ml) werden 13,1 g = 81%d.Th. des Reaktionsproduktes erhalten. Außerdem werden 8,51 g 5-Fluor-2,3-dihydrobenzofuran (Destillat) als farblose Flüssigkeit zurückgewonnen Schmelzpunkt: 72,4°C
¹H-NMR (600 MHz, CDCl₃); δ = 1.21 (t, 3H), 1.35 (s, 3H), 1.40 (s, 3H), 2.43 (d, 1 H), 2.745 (d, 1 H), 3.15 (m, 2H), 3.56 (sbr, 1 H), 3.73 (dq, 1 H), 4.125 (dq, 1 H), 4.58 (t, 2H), 6.68 (dd, 1 H), 6.77 (dm, 1H).Der erhaltene Ester kann dann als Racemat oder auch als reines Enantiomer, wie beispielsweise in WO 03/082827 beschrieben, weiter umgesetzt werden zu den Verbindungen der allgemeinen Formel IIa und IIb.

## Patentansprüche

1. Verbindungen der allgemeinen Formeln (IIa) oder (IIb) 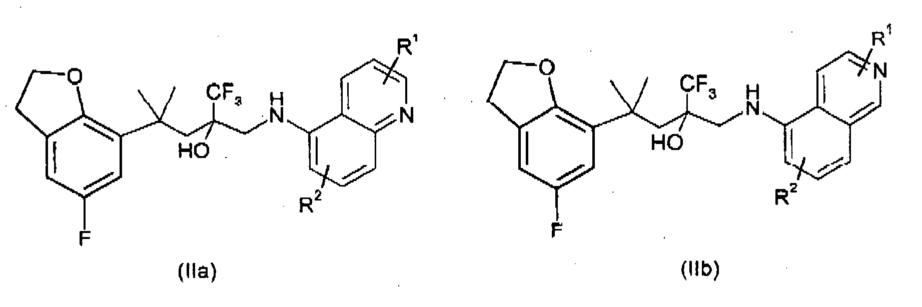 worin
R¹ und R² unabhängig voneinander ein Wasserstoffatom, eine C₁₋₃-Alkylgruppe, ein Halogenatom, eine Cyano-, eine C₁₋₃-Alkoxy- oder eine Hydroxygruppe bedeuten,
sowie deren Racemate oder getrennt vorliegenden Stereoisomere und deren physiologisch verträgliche Salze.

2. Verbindungen der allgemeinen Formeln (IIa) oder (IIb) gemäß Anspruch 1, worin R¹ und R² unabhängig voneinander ein Wasserstoffatom, ein Chloratom, eine Methyl- oder eine Hydroxygruppe.

3. Verbindung gemäß Anspruch 1, nämlich
5-[4-(5-Fluor-2,3-dihydrobenzofuran-7-yl)-2-hydroxy-4-methyl-2-trifluormethylpentylamino]-2-methylchinolin,
5-[4-(5-Fluor-2,3-dihydrobenzofuran-7-yl)-2-hydroxy-4-methyl-2-trifluormethylpentylamino]-1-methylisochinolin,
5-[4-(5-Fluor-2,3-dihydrobenzofuran-7-yl)-2-hydroxy-4-methyl-2-trifluormethylpentylamino]isochinol-1(2H)-on
5-[4-(5-Fluor-2,3-dihydrobenzofuran-7-yl)-2-hydroxy-4-methyl-2-trifluormethylpentylamino]-2,6-dimethylchinolin
5-[4-(5-Fluor-2,3-dihydrobenzofuran-7-yl)-2-hydroxy-4-methyl-2-trifluormethylpentylamino]-6-chlor-2-methylchinolin
5-[4-(5-Fluor-2,3-dihydrobenzofuran-7-yl)-2-hydroxy-4-methyl-2-trifluormethylpentylamino]isochinolin
5-[4-(5-Fluor-2,3-dihydrobenzofuran-7-yl)-2-hydroxy-4-methyl-2-trifluormethylpentylamino]chinolin
5-[4-(2,3-Dihydro-5-fluoro-7-benzofuranyl)-2-hydroxy-4-methyl-2-trifluormethylpentylamino]chinolin-2[1 H]-on,
6-Fluor-5-[4-(5-fluor-2,3-dihydrobenzofuran-7-yl)-2-hydroxy-4-methyl-2-trifluormethyl-pentylamino]-2-methylchinolin,
8-Fluor-5-[4-(5-fluor-2,3-dihydrobenzofuran-7-yl)-2-hydroxy-4-methyl-2-trifluormethyl-pentylamino]-2-methylchinolin
oder
5-[4-(5-Fluor-2,3-dihydrobenzofuran-7-yl)-2-hydroxy-4-methyl-2-trifluormethylpentylamino]-2-methylisochinol-1(2H)-on.

4. (+)-Enantiomere der Verbindungen gemäß Anspruch 3.

5. (-)-Enantiomere der Verbindungen gemäß der Anspruch 3.

6. Verfahren zur Herstellung von Verbindungen der allgemeinen Formeln IIa und IIb, **dadurch gekennzeichnet, dass** Isobuten unter Verwendung eines lewissauren Katalysators mit Trifluoralkylpyruvat zur Verbindung der allgemeinen Formel XII 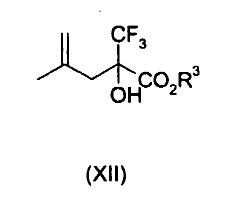 wobei R³ C₁-C₅-Alkyl bedeutet, umgesetzt wird, die, wenn gewünscht einer Enantiomerentrennung unterworfen werden kann, und anschließend als chirale Verbindung oder als Racemat mit 5-Fluor-2,3-dihydrobenzofuran 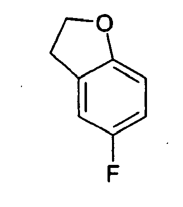 in einer Friedel Crafts Alkylierungsreaktion zu Verbindungen der allgemeinen Formel X 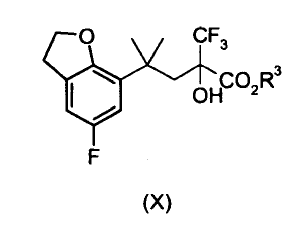 umgesetzt wird, die wenn gewünscht, einer Enantiomerentrennung unterworfen werden kann und anschließend
die Verbindung der allgemeinen Formel X - als chirale Verbindung oder als Racemat -
entweder
zum Alkohol XI 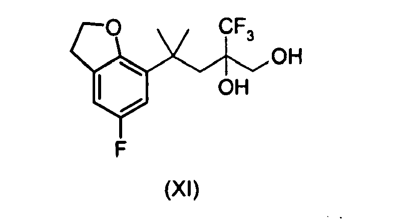 reduziert und anschließend zum Aldehyd (III) 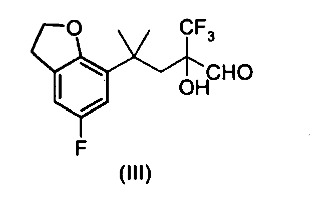 oxidiert wird
oder
die Verbindung X direkt zum Aldehyd III reduziert werden, wobei der erhaltene Aldehyd (III) anschließend zu Verbindungen der allgemeinen Formel IIa oder IIb umgesetzt wird, indem unter Bedingungen der reduktiven Aminierung, gegebenenfalls in zwei Stufen, mit einem 5-Aminochinolinderivat 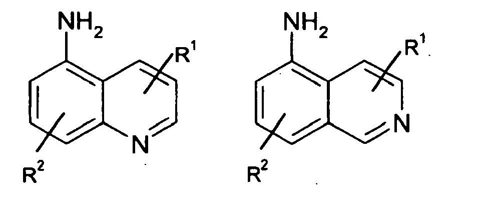 oder mit einem 5-Aminoisochinolinderivat, worin R¹ und R² die in Anspruch 1 angegebenen Bedeutungen haben, umgesetzt wird.

7. Verfahren gemäß Anspruch 6, **dadurch gekennzeichnet, dass** die Enantiomerentrennung auf einer beliebigen geeigneten Stufe mit Hilfe von Säulenchromatographie an einer chiralen Phase erfolgt.

8. Verfahren gemäß Anspruch 6, **dadurch gekennzeichnet, dass** man eine geeignete racemische Zwischenstufe aus der Synthese mit Hilfe eines chiralen Hilfsstoffes entweder in die diastereomeren Salze oder in Diastereomere überführt, anschließend die Diastereomerentrennung durchführt und das chirale Hilfsreagenz wieder abspaltet.

9. Verfahren gemäß Anspruch 7, wobei die Enantiomerentrennung auf der Stufe der Verbindung der allgemeinen Formeln III, X oder X' erfolgt.

10. Verfahren gemäß Anspruch 8, wobei der chirale Hilfsstoff eine chirale Base, ein chirales Diol, ein chiraler Alkohol oder eine chirale Säure ist.

11. Verfahren zur Herstellung der Verbindungen der allgemeinen Formel IIa und IIb **dadurch gekennzeichnet, dass** der Aldehyd (III) 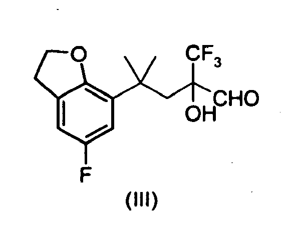 in chiraler oder racemischer Form unter Bedingungen der reduktiven Aminierung, gegebenenfalls in zwei Stufen, mit einem 5-Aminochinolinderivat 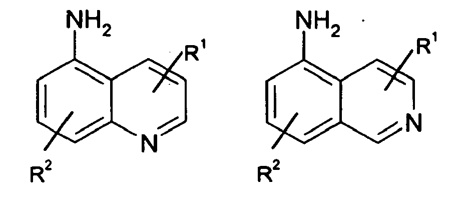 oder mit einem 5-Aminoisochinolinderivat, worin R¹ und R² die in Anspruch 1 angegebenen Bedeutungen haben, umgesetzt wird.

12. Verfahren zur Herstellung von Verbindungen der allgemeinen Formeln IIa und IIb gemäß Anspruch 11, **dadurch gekennzeichnet, dass** die Verbindung der allgemeinen Formel III erhalten wird
entweder
durch Reduktion der Verbindung der allgemeinen Formel X - eingesetzt als chirale Verbindung oder als Racemat - 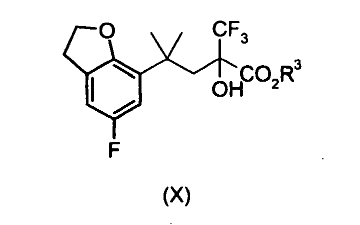 worin R³ C₁-C₅-Alkyl bedeutet,
nach dem Fachmann bekannten Methoden zum Alkohol XI 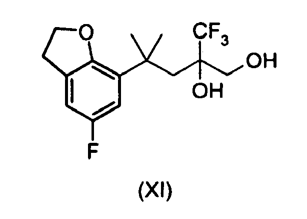 und anschließender Oxidation nach ebenfalls dem Fachmann bekannten Methoden zum Aldehyd (III)
oder
durch Reduktion die Verbindung X - eingesetzt als chirale Verbindung oder als Racemat - nach dem Fachmann bekannter Methode direkt zum Aldehyd III 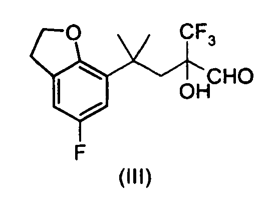 umgesetzt wird.

13. Verfahren zur Herstellung von Verbindungen der allgemeinen Formeln IIa und IIb gemäß Anspruch 12, **dadurch gekennzeichnet, dass** die Verbindung der allgemeinen Formel X erhalten wird,
indem eine Verbindung der allgemeinen Formel XII 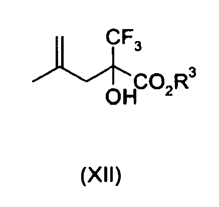 wobei R³ C₁-C₅-Alkyl bedeutet, die, wenn gewünscht einer Enantiomerentrennung unterworfen werden kann, anschließende als chirale Verbindung oder als Racemat mit 5-Fluor-2,3-dihydrobenzofuran 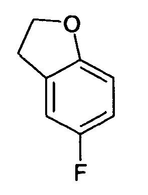 in einer Friedel Crafts Alkylierungsreaktion zu Verbindungen der allgemeinen Formel X 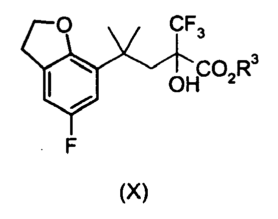 umgesetzt wird, die, wenn gewünscht, einer Enantiomerentrennung unterworfen werden kann.

14. Verfahren zur Herstellung von Verbindungen der allgemeinen Formeln IIa und IIb gemäß Anspruch 13, **dadurch gekennzeichnet, dass** die Verbindung der allgemeinen Formel XII erhalten wird,
indem Isobuten unter Verwendung eines lewissauren Katalysators mit Trifluoralkylpyruvat zur Verbindung der allgemeinen Formel XII 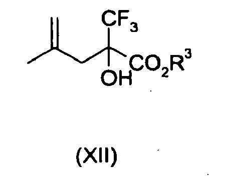 wobei R³ C₁-C₅-Alkyl bedeutet, umgesetzt wird, die, wenn gewünscht, einer Enantiomerentrennung unterworfen wird.

15. Verfahren gemäß Anspruch 6 oder 14, **dadurch gekennzeichnet, dass** der lewissaure Katalysator chiral ist.

16. Verfahren gemäß Anspruch 6 oder 11, **dadurch gekennzeichnet, dass** die Verbindung der allgemeinen Formel XII aus Anspruch 6 oder die Verbindung der allgemeinen Formel III aus Anspruch 11 chiral eingesetzt werden.

17. Verfahren gemäß mindestens einem der Ansprüche 12-14, **dadurch gekennzeichnet, dass** die jeweils erhaltenen Zwischenprodukte einer Enantiomeren- oder nach Umsetzen mit chiralen Hilfsstoffen einer Diastereomerentrennung unterworfen werden.

18. Verbindung der Formel III 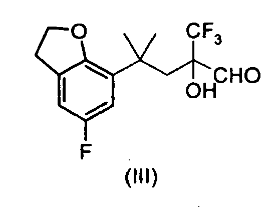 in chiraler oder racemischer Form.

19. Verwendung der Verbindungen mindestens einem der Ansprüche 1-5 zur Herstellung von Arzneimitteln.

20. Verwendung der Verbindungen gemäß mindestens einem der Ansprüche 1-5 zur Herstellung eines Arzneimittels zur Behandlung von entzündlichen Erkrankungen.

21. Verwendung der Verbindungen gemäß mindestens einem der Ansprüche 1-5 zur Herstellung von Arzneimitteln, die lokal verabreicht werden.

22. Pharmazeutische Präparate enthaltend mindestens eine Verbindung nach Anspruch 1 bis 5 oder deren Gemische sowie pharmazeutisch verträgliche Träger.

## Claims

1. Compounds of general formula (IIa) or (IIb) 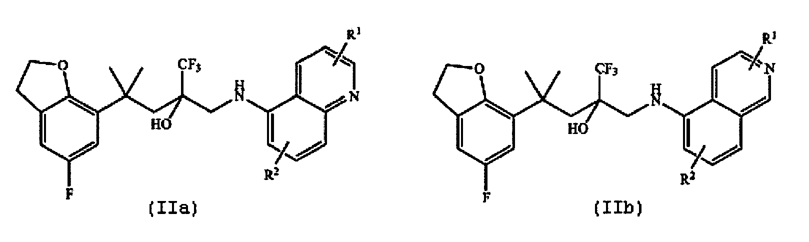 in which
R¹ and R² independently of one another, mean a hydrogen atom, a C₁₋₃-alkyl group, a halogen atom, a cyano group, a C₁₋₃-alkoxy group or a hydroxy group,
as well as their racemates or separately present stereoisomers thereof and their physiologically compatible salts thereof.

2. Compounds of general formula (IIa) or (IIb) according to Claim 1, in which R¹ and R², independently of one another, mean a hydrogen atom, a chlorine atom, a methyl or a hydroxy group.

3. Compound according to Claim 1, which is
5-[4-(5-fluoro-2,3-dihydrobenzofuran-7-yl)-2-hydroxy-4-methyl-2-trifluoromethyl-pentylamino]-2-methylquinoline
5-[4-(5-fluoro-2,3-dihydrobenzofuran-7-yl)-2-hydroxy-4-methyl-2-trifluoromethyl-pentylamino]-1-methylisoquinoline
5-[4-(5-fluoro-2,3-dihydrobenzofuran-7-yl)-2-hydroxy-4-methyl-2-trifluoromethyl-pentylamino]isoquinol-1(2H)-one
5-[4-(5-fluoro-2,3-dihydrobenzofuran-7-yl)-2-hydroxy-4-methyl-2-trifluoromethyl-pentylamino]-2,6-dimethylquinoline
5-[4-(5-fluoro-2,3-dihydrohenzofuran-7-yl)-2-hydroxy-4-methyl-2-trifluoromethyl-pentylamino]-6-chloro-2-methylquinoline
5-[4-(5-fluoro-2,3-dihydrobenzofuran-7-yl)-2-hydroxy-4-methyl-2-trifluoromethyl-pentylamino]isoquinoline
5-[4-(5-fluoro-2,3-dihydrobenzofuran-7-yl)-2-hydroxy-4-methyl-2-trifluoromethyl-pentylamino]quinoline
5-[4-(2,3-dihydro-5-fluoro-7-benzofuranyl)-2-hydroxy-4-methyl-2-trifluoromethyl-pentylamino]quinolin-2[1H]-one
6-fluoro-5-[4-(5-fluoro-2,3-dihydrobenzofuran-7-yl)-2-hydroxy-4-methyl-2-trifluoromethyl-pentylamino]-2-methylquinoline
8-fluoro-5-[4-(5-fluoro-2,3-dihydrobenzofuran-7-yl)-2-hydroxy-4-methyl-2-trifluoromethyl-pentylamino]-2-methylquinoline
or
5-[4-(5-fluoro-2,3-dihydrobenzofuran-7-yl)-2-hydroxy-4-methyl-2-trifluoromethyl-pentylamino]-2-methylisoquinol-1(2H)-one.

4. (+)-enantiomers of the compounds according to Claim 3.

5. (-)-enantiomers of the compounds according to Claim 3.

6. Process for the production of compounds of general formulas IIa and IIb, **characterized in that** isobutene is reacted with use of a Lewis acid catalyst with trifluoroalkyl pyruvate to form the compound of general formula XII 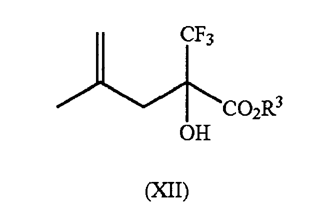 wherein R³ means C₁-C₅-alkyl, which can be subjected, if desired, to an enantiomer separation and then is reacted as a chiral compound or as a racemate with 5-fluoro-2,3-dihydrobenzofuran 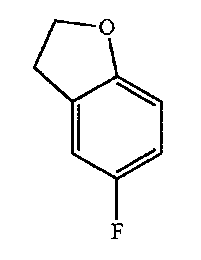 in a Friedel Crafts alkylation reaction to form compounds of general formula X 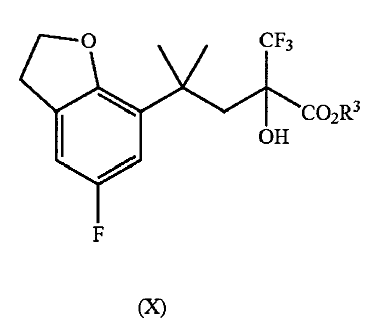 which, if desired, can be subjected to an enantiomer separation, and then
the compound of general formula X - as a chiral compound or as a racemate -
is either
reduced to form alcohol XI 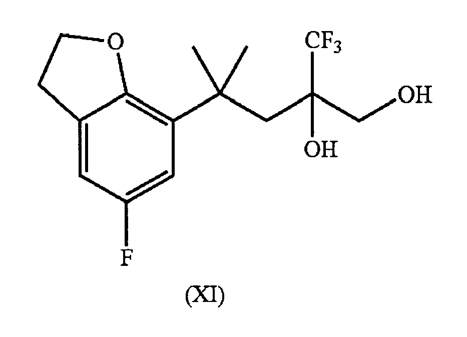 and then oxidized to form aldehyde (III) 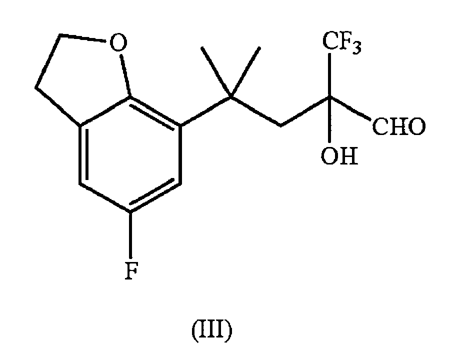 or
compound X is reduced directly to form aldehyde III and the aldehyde (III) obtained is then reacted to form compounds of general formula IIa or IIb by being reacted under the conditions of reductive amination, optionally in two stages, with a 5-aminoquinoline derivative 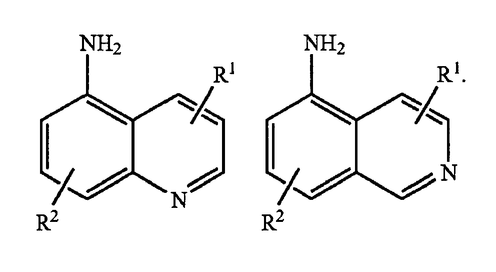 or with a 5-aminoisoquinoline derivative, in which R¹ and R² have the meanings indicated in Claim 1.

7. Process according to Claim 6, **characterized in that** the enantiomer separation is carried out in any suitable stage using column chromatography on a chiral phase.

8. Process according to Claim 6, **characterized in that** a suitable racemic intermediate stage from the synthesis is converted using a chiral adjuvant either into the diastereomer salts or into diastereomers, then the diastereomer separation is performed, and the chiral auxiliary reagent is cleaved again.

9. Process according to Claim 7, wherein the enantiomer separation is carried out at the stage of the compound of general formula III, X or XI.

10. Process according to Claim 8, wherein the chiral adjuvant is a chiral base, chiral diol, chiral alcohol or chiral acid.

11. Process for the production of the compounds of general formulas IIa and IIb, **characterized in that** the aldehyde (III) 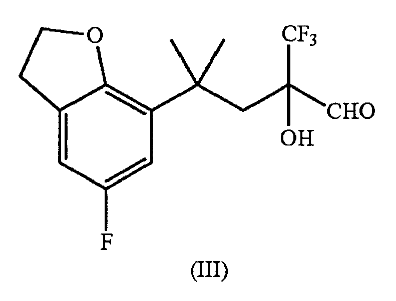 in chiral or racemic form is reacted under conditions of reductive amination, optionally in two stages, with a 5-aminoquinoline derivative 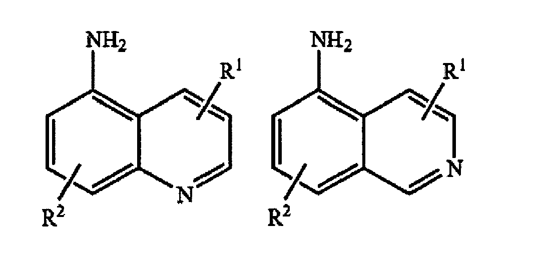 or with a 5-aminoisoquinoline derivative, in which R¹ and R² have the meanings that are indicated in Claim 1.

12. Process for the production of compounds of general formulas IIa and IIb according to Claim 11, **characterized in that** the compound of general formula III is obtained
either
by reduction of the compound of general formula X - used as a chiral compound or as a racemate - 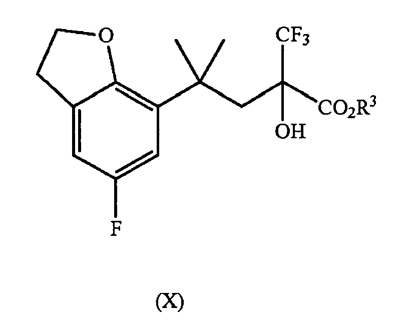 in which R³ means C₁-C₅-alkyl,
according to methods that are known to one skilled in the art to form alcohol XI 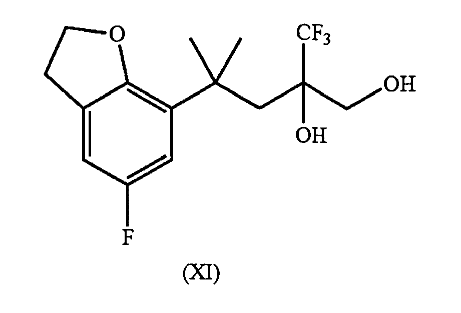 and subsequent oxidation according to methods that are likewise known to one skilled in the art to form aldehyde (III)
or
by reduction the compound X - used as a chiral compound or as a racemate - according to the method that is known to one skilled in the art is reacted directly to form aldehyde III

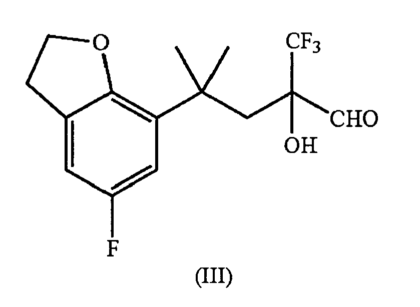

13. Process for the production of compounds of general formulas IIa and IIb according to Claim 12, **characterized in that** the compound of general formula X is obtained by a compound of general formula XII 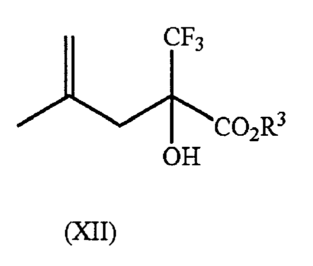 wherein R³ means C₁-C₅-alkyl, which can be subjected, if desired, to an enantiomer separation, then being reacted as a chiral compound or as a racemate with 5-fluoro-2,3-dihydrobenzofuran 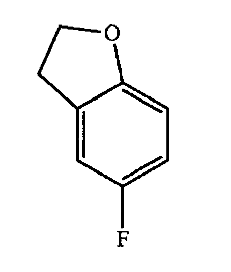 in a Friedel-Crafts alkylation reaction to form compounds of general formula X 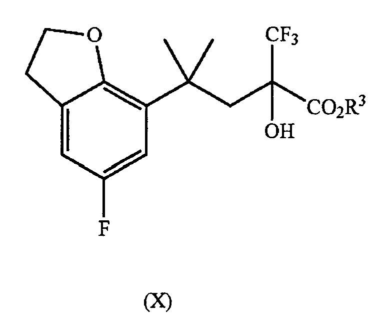 which can be subjected, if desired, to an enantiomer separation.

14. Process for the production of compounds of general formulas IIa and IIb according to Claim 13, **characterized in that** the compound of general formula XII is obtained by
isobutene being reacted with use of a Lewis acid catalyst with trifluoroalkyl pyruvate to form the compound of general formula XII 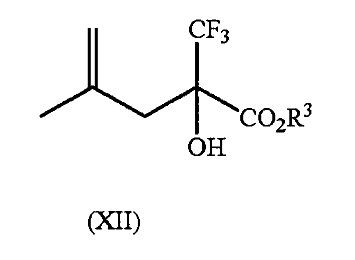 wherein R³ means C₁-C₅-alkyl, which, if desired, is subjected to an enantiomer separation.

15. Process according to Claim 6 or 14, **characterized in that** the Lewis acid catalyst is chiral.

16. Process according to Claim 6 or 11, **characterized in that** the compound of general formula XII from Claim 6 or the compound of general formula III from Claim 11 are used in a chiral manner.

17. Process according to at least one of Claims 12 - 14, **characterized in that** the intermediate products that are obtained in each case are subjected to an enantiomer separation or, after reaction with chiral adjuvants, to a diastereomer separation.

18. Compound of Formula III, 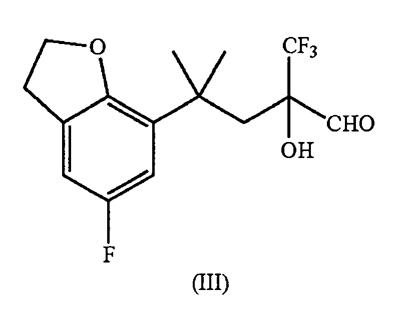 in chiral or racemic form.

19. Use of the compounds according to at least one of Claims 1 - 5 for the production of pharmaceutical agents.

20. Use of the compounds according to at least one of Claims 1 - 5 for the production of a pharmaceutical agent for the treatment of inflammatory diseases.

21. Use of the compounds according to at least one of Claims 1 - 5 for the production of pharmaceutical agents that are administered locally.

22. Pharmaceutical preparations that contain at least one compound according to Claims 1 to 5 or mixtures thereof as well as pharmaceutically compatible vehicles.

## Revendications

1. Composés de formules générales (IIa) et (IIb) 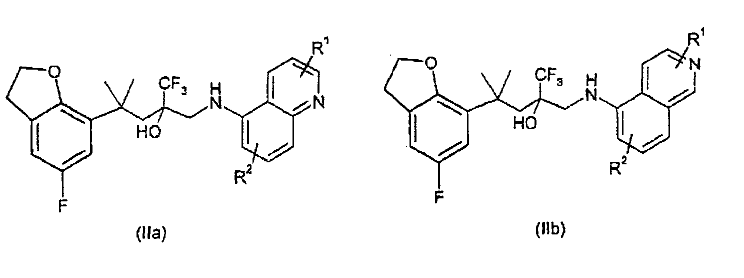 dans lesquelles
R¹ et R² représentent, indépendamment l'un de l'autre, un atome d'hydrogène, un groupe alkyle en C₁-C₃, un atome d'halogène, un groupe cyano, un groupe alcoxy en C₁-C₃ ou un groupe hydroxy,
ainsi que leurs racémates ou stéréoisomères présents séparément et leurs sels physiologiquement acceptables.

2. Composés de formules générales (IIa) et (IIb) selon la revendication 1, dans lesquels R¹ et R² représentent, indépendamment l'un de l'autre, un atome d'hydrogène, un atome de chlore, un groupe méthyle ou un groupe hydroxy.

3. Composé selon la revendication 1, à savoir
5-[4-(5-fluoro-2,3-dihydrobenzofurann-7-yl)-2-hydroxy-4-méthyl-2-trifluorométhyl-pentylamino]-2-méthylquinoléine,
5-[4-(5-fluoro-2,3-dihydrobenzofurann-7-yl)-2-hydroxy-4-méthyl-2-trifluorométhyl-pentylamino]-1-méthylisoquinoléine,
5-[4-(5-fluoro-2,3-dihydrobenzofurann-7-yl)-2-hydroxy-4-méthyl-2-trifluorométhyl-pentylamino]-isoquinol-1(2H)-one,
5-[4-(5-fluoro-2,3-dihydrobenzofurann-7-yl)-2-hydroxy-4-méthyl-2-trifluorométhyl-pentylamino]-2,6-diméthylquinoléine,
5-[4-(5-fluoro-2,3-dihydrobenzofurann-7-yl)-2-hydroxy-4-méthyl-2-trifluorométhyl-pentylamino]-6-chloro-2-méthylquinoléine,
5-[4-(5-fluoro-2,3-dihydrobenzofurann-7-yl)-2-hydroxy-4-méthyl-2-trifluorométhyl-pentylamino]-isoquinoléine,
5-[4-(5-fluoro-2,3-dihydrobenzofurann-7-yl)-2-hydroxy-4-méthyl-2-trifluorométhyl-pentylamino]-quinoléine,
5-[4-(2,3-dihydro-5-fluoro-7-benzofurannyl)-2-hydroxy-4-méthyl-2-trifluorométhyl-pentylamino]-quinolin-2(1H)-one,
6-fluoro-5-[4-(5-fluoro-2,3-dihydrobenzofurann-7-yl)-2-hydroxy-4-méthyl-2-trifluorométhylpentylamino]-2-méthylquinoléine,
8-fluoro-5-[4-(5-fluoro-2,3-dihydrobenzofurann-7-yl)-2-hydroxy-4-méthyl-2-trifluorométhylpentylamino]-2-méthylquinoléine,
ou
5-[4-(5-fluoro-2,3-dihydrobenzofurann-7-yl)-2-hydroxy-4-méthyl-2-trifluorométhyl-pentylamino]-2-méthylisoquinol-1(2H)-one.

4. Énantiomères (+) des composés selon la revendication 3.

5. Énantiomères (-) des composés selon la revendication 3.

6. Procédé pour la préparation de composés de formules générales IIa et IIb, **caractérisé en ce qu'**on fait réagir de l'isobutène, en utilisant un catalyseur de type acide de Lewis, avec un pyruvate de trifluoroalkyle, pour aboutir au composé de formule générale XII 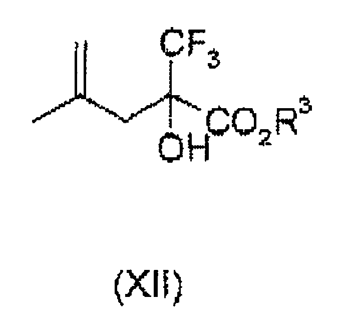 dans laquelle R³ représente un groupe alkyle en C₁-C₅, qui peut, si on le désire, être soumis à une séparation d'énantiomères, et ensuite on le fait réagir, sous forme de composé chiral ou de racémate, avec du 5-fluoro-2,3-dihydrobenzofuranne 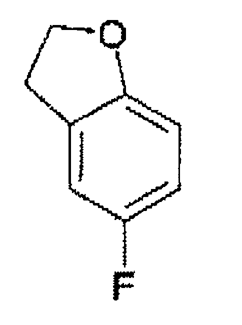 dans une réaction d'alkylation de Friedel et Crafts pour aboutir à un composé de formule générale X 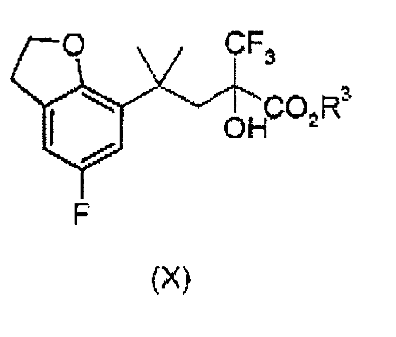 qui peut être soumis, si on le désire, à une séparation d'énantiomères, et ensuite
soit
on réduit le composé de formule générale X - sous forme de composé chiral ou de racémate - en l'alcool XI 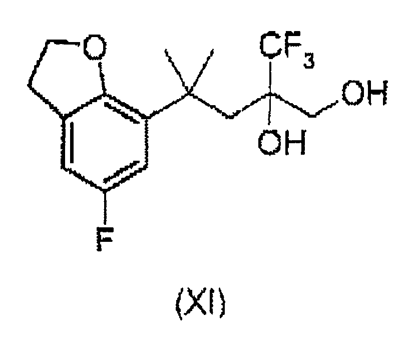 et ensuite on l'oxyde en l'aldéhyde (III) 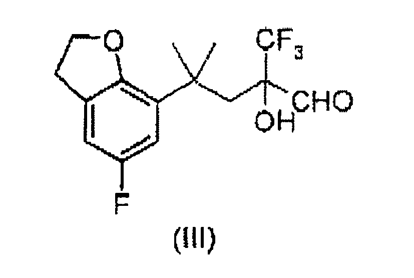 soit
on réduit directement le composé X en l'aldéhyde III, et on convertit ensuite l'aldéhyde (III) obtenu en composés de formule générale IIa ou IIb en le faisant réagir, dans des conditions de l'amination réductrice, éventuellement en deux étapes, avec un dérivé de 5-aminoquinoléine 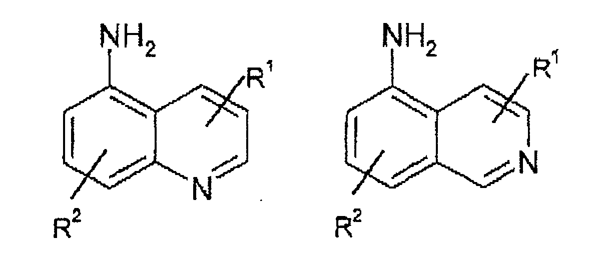 ou avec un dérivé de 5-amino-isoquinoléine, R¹ et R² ayant les significations indiquées dans la revendication 1.

7. Procédé selon la revendication 6, **caractérisé en ce que** la séparation d'énantiomères s'effectue à un stade convenable quelconque, à l'aide de chromatographie sur colonne sur une phase chirale.

8. Procédé selon la revendication 6, **caractérisé en ce qu'**on convertit un produit intermédiaire racémique approprié de la synthèse, à l'aide d'un composé auxiliaire chiral, soit en les sels diastéréoisomères, soit en diastéréoisomères, puis on effectue la séparation des diastéréoisomères et on sépare à nouveau le réactif auxiliaire chiral.

9. Procédé selon la revendication 7, dans lequel la séparation des énantiomères s'effectue au stade du composé de formule générale III, X ou XI.

10. Procédé selon la revendication 8, dans lequel le composé auxiliaire chiral est une base chirale, un diol chiral, un alcool chiral ou un acide chiral.

11. Procédé pour la préparation des composés de formules générales IIa et IIb, **caractérisé en ce qu'**on fait réagir l'aldéhyde (III) 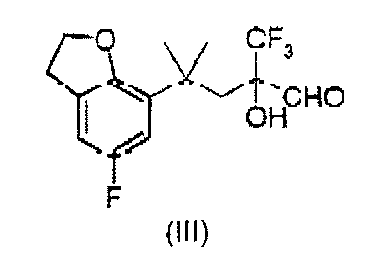 sous forme chirale ou racémique, dans des conditions de l'amination réductrice, éventuellement en deux étapes, avec un dérivé de 5-aminoquinoléine 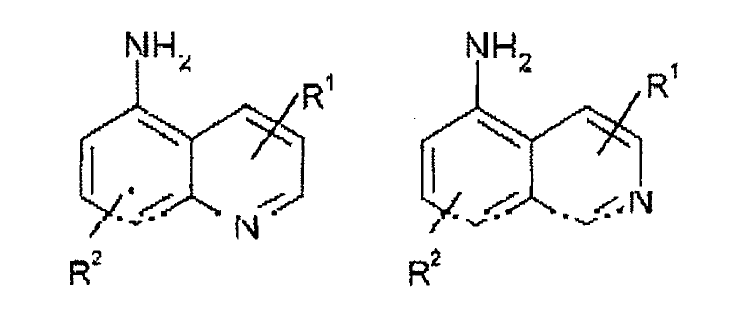 ou avec un dérivé de 5-amino-isoquinoléine, où R¹ et R² ont les significations indiquées dans la revendication 1.

12. Procédé pour la préparation de composés de formules générales IIa et IIb selon la revendication 11, **caractérisé en ce qu'**on obtient le composé de formule générale III soit
par réduction du composé de formule générale X - utilisé sous forme de composé chiral ou de racémate - 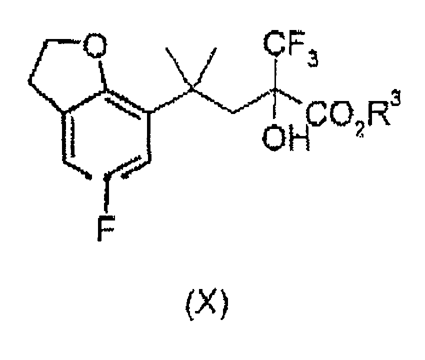 dans laquelle R³ représente un groupe alkyle en C₁-C₅
selon des méthodes connues de l'homme de métier, pour aboutir à l'alcool XI 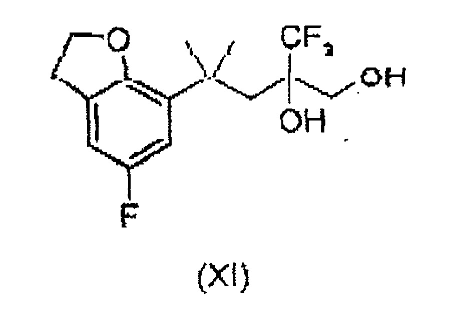 et oxydation subséquente, selon des méthodes également connues de l'homme de métier, en l'aldéhyde (III)
soit
on convertit par réduction directement le composé X - utilisé sous forme de composé chiral ou de racémate - selon des méthodes connues de l'homme de métier, en l'aldéhyde III

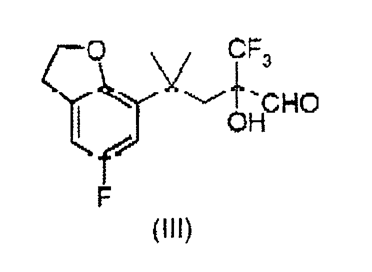

13. Procédé pour la préparation de composés de formules générales IIa et IIb selon la revendication 12, **caractérisé en ce qu'**on obtient le composé de formule générale X
en faisant réagir un composé de formule générale XII 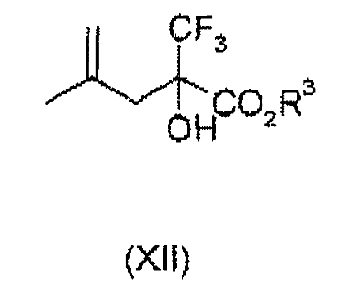 dans laquelle R³ représente un groupe alkyle en C₁-C₅, qui, si on le désire, peut être soumis à une séparation d'énantiomères, ensuite sous forme de composé chiral ou de racémate, avec du 5-fluoro-2,3-dihydrobenzofuranne 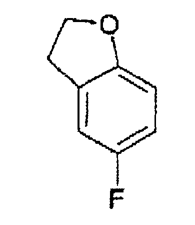 dans une réaction d'alkylation de Friedel et Crafts, pour aboutir à un composé de formule générale X 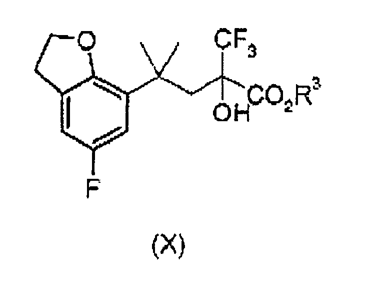 qui, si on le désire, peut être soumis à une séparation d'énantiomères.

14. Procédé pour la préparation de composés de formules générales IIa et IIb selon la revendication 13, **caractérisé en ce qu'**on obtient le composé de formule générale XII
en faisant réagir de l'isobutène, avec utilisation d'un catalyseur de type acide de Lewis, avec un pyruvate de trifluoroalkyle, pour aboutir à un composé de formule générale XII 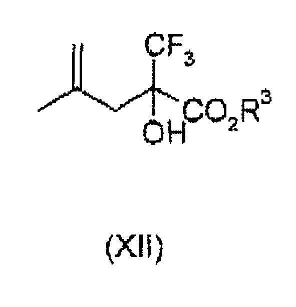 dans laquelle R³ représente un groupe alkyle en C₁-C₅, qui, si on le désire, est soumis à une séparation d'énantiomères.

15. Procédé selon la revendication 6 ou 14, **caractérisé en ce que** le catalyseur de type acide de Lewis est chiral.

16. Procédé selon la revendication 6 ou 11, **caractérisé en ce que** le composé de formule générale XII de la revendication 6 ou le composé de formule générale III de la revendication 11 sont utilisés sous forme chirale.

17. Procédé selon au moins l'une des revendications 12 à 14, **caractérisé en ce que** les produits intermédiaires obtenus dans chaque cas sont soumis à une séparation d'énantiomères ou, après réaction avec des composés auxiliaires chiraux, à une séparation de diastéréoisomères.

18. Composé de formule III 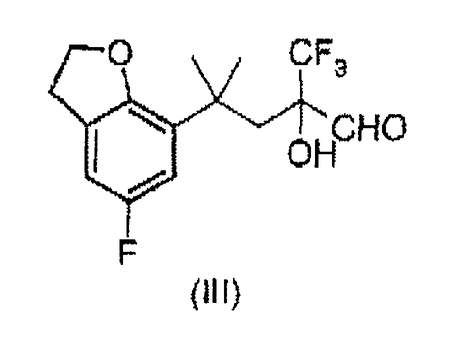 sous forme racémique ou chirale.

19. Utilisation des composés selon au moins l'une des revendications 1 à 5, pour la fabrication de médicaments.

20. Utilisation des composés selon au moins l'une des revendications 1 à 5, pour la fabrication de médicaments destinés au traitement de maladies inflammatoires.

21. Utilisation des composés selon au moins l'une des revendications 1 à 5, pour la fabrication de médicaments qui sont administrés localement.

22. Préparations pharmaceutiques contenant au moins un composé selon l'une quelconque des revendications 1 à 5, ou de mélanges de ceux-ci, ainsi que des véhicules pharmaceutiquement acceptables.
